(19) **Europäisches Patentamt European Patent Office Office européen des brevets**

(11) **EP 4 772 539 A1**

(12) **EUROPEAN PATENT APPLICATION**
published in accordance with Art. 153(4) EPC

(43) Date of publication:
08.07.2026 Bulletin 2026/28

(21) Application number: 24858721.4

(22) Date of filing: 30.08.2024

(51) International Patent Classification (IPC):
*C07K 16/46* (2006.01)     *A61K 39/395* (2006.01)
*A61K 39/00* (2006.01)     *A61P 35/00* (2006.01)

(52) Cooperative Patent Classification (CPC):
A61K 39/00; A61K 39/395; A61P 35/00;
C07K 16/46

(86) International application number:
PCT/CN2024/115779

(87) International publication number:
WO 2025/045190 (06.03.2025 Gazette 2025/10)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 31.08.2023 CN 202311110063

(71) Applicants:
• Jiangsu Hengrui Pharmaceuticals Co., Ltd.
Lianyungang, Jiangsu 222047 (CN)
• Shanghai Shengdi Pharmaceutical Co., Ltd
Shanghai 201210 (CN)

(72) Inventors:
• YAN, Hong
Shanghai 201210 (CN)
• BAO, Luyao
Shanghai 201210 (CN)
• ZHANG, Wei
Shanghai 201210 (CN)
• CHEN, Simeng
Shanghai 201210 (CN)
• YUAN, Jimin
Shanghai 201210 (CN)
• LIAO, Cheng
Lianyungang, Jiangsu 222047 (CN)

(74) Representative: **Potter Clarkson**
**Chapel Quarter**
**Mount Street**
**Nottingham NG1 6HQ (GB)**

Remarks:
The complete document including Reference
Table(s) and the Sequence Listing(s) can be
downloaded from the EPO website

(54) **CTLA4/TIGIT BINDING PROTEIN AND PHARMACEUTICAL USE THEREOF**

(57) Provided are a CTLA4/TIGIT binding protein and the pharmaceutical use thereof. Specifically, the present invention relates to an anti-CTLA4/TIGIT antibody and a method for treating cancers and the pharmaceutical use thereof.

EP 4 772 539 A1

## Description

The present application claims the right of priority for the Chinese patent application CN 202311110063.8 filed on August 31, 2023.

## Technical Field

The present disclosure relates to a protein that binds to CTLA4 and TIGIT, such as an anti-CTLA4/TIGIT antibody, and the use thereof in treating cancer.

## Background Art

During the anti-tumour immune response, T cell activation requires two signals. The first signal is antigen-specific and is initiated by the T cell receptor (TCR) complex recognising the antigen peptide-MHC complex on the surface of APC cells; and the second signal is a co-stimulatory signal that requires the involvement of co-stimulatory molecules such as CD28. Upon binding to ligands CD80/CD86, CD28 further activates T cells for promoting the maturation and proliferation of the T cells, thereby initiating immune responses (Nat Rev Immunol. 2013 Apr; 13 (4):227-42). Regulatory T cells (also known as Treg cells) are an important subset of T cells with immunosuppressive functions. Treg cells exert immunosuppressive functions mainly by cell-contact-dependent inhibition (e.g., expressing inhibitory receptors that interact with ligands on target cell surfaces) and contact-independent inhibition (e.g., secreting various immunosuppressive cytokines such as IL-10) (Nat Immunol. 2019 Feb; 20 (2): 218-231).

CTLA4 protein (cytotoxic T lymphocyte-associated antigen-4, also known as CD152) is an immune checkpoint molecule primarily expressed on the surface of activated T cells and Treg cells. It competes with CD28 for binding to ligands CD80 and CD86, with CTLA4 having the higher affinity, thereby exerting an immunosuppressive effect.

CTLA4 monoclonal antibodies approved for market include Ipilimumab and Tremelimumab monoclonal antibodies. Such two antibodies themselves may induce CLTA4-characteristic toxicities clinically, including dermatitis, diarrhoea, and enteritis (Oncogene. 2015 Oct; 34 (43): 5411-7.; J Transl Med. 2012 Nov 21; 10: 236.), indicating that the toxicity of CTLA4 arises from two aspects: ADCC-mediated depletion of peripheral Treg cells and the inhibition of CTLA4 signalling in peripheral blood, which disrupts peripheral immune balance and results in immunotoxicity. Therefore, developing CTLA4 antibodies that mitigate side effects without compromising efficacy holds significant clinical application value.

TIGIT protein (T cell immunoreceptor with Ig and ITIM domains, also known as VSIG9) is an immune checkpoint molecule that is primarily expressed on activated T cells. Ligands for TIGIT include CD155 and CD112. CD155 has a higher affinity for TIGIT, while CD112 has a weaker affinity for TIGIT. Meanwhile, CD155 and CD112 are ligands for the co-stimulatory molecule CD226. TIGIT competes with CD226 for binding to CD155 and CD112 on the surface of tumour cells or APC cells, thereby exerting an immunosuppressive effect (Trends Immunol. 2017 Jan; 38 (1): 20-28.). TIGIT antibodies exhibit a good safety profile in the clinic (Biomedicines. 2021 Sep; 9 (9): 1277.).

The present disclosure provides an anti-CTLA4/TIGIT antibody with a new structure. Through structural design, the antibody can selectively target intratumoural Treg cells co-expressing the target proteins via an avidity effect. The antibody exhibits excellent tumour growth inhibition and killing effects, outstanding potential to be manufactured as a drug, and high potential for clinical safety.

## Summary of the Invention

The present disclosure provides a CTLA4/TIGIT binding protein, a nucleic acid encoding same, a vector thereof, a host cell thereof, a pharmaceutical composition thereof, a method for treating cancer, depleting Treg cells, or enhancing T cells using same, and the related pharmaceutical use thereof.

CTLA4/TIGIT binding protein

The present disclosure provides a CTLA4/TIGIT binding protein, which comprises a first binding domain that specifically binds to CTLA4 and a second binding domain that specifically binds to TIGIT, and is capable of specifically binding to CTLA4 and TIGIT simultaneously or separately.

Regarding the first binding domain that specifically binds to the CTLA4:

In some embodiments, the first binding domain that specifically binds to CTLA4 in the CTLA4/TIGIT binding protein comprises a heavy chain variable region (VH1) and a light chain variable region (VL1), wherein the VH1 comprises HCDR1, HCDR2, and HCDR3 in an amino acid sequence as set forth in SEQ ID NO: 13, and the VL1 comprises LCDR1, LCDR2, and LCDR3 in an amino acid sequence as set forth in SEQ ID NO: 14.

The CDRs are defined according to the Kabat, IMGT, Chothia, AbM, or Contact numbering system. In some specific embodiments, the CDRs are defined according to the Kabat numbering system.

In some embodiments, the first binding domain that specifically binds to CTLA4 in the CTLA4/TIGIT binding protein comprises a heavy chain variable region (VH1) and a light chain variable region (VL1), wherein the VH1 comprises HCDR1, HCDR2, and HCDR3 as set forth in SEQ ID NOs: 1, 2, and 3, respectively, and the VL1 comprises LCDR1, LCDR2, and LCDR3 as set forth in SEQ ID NOs: 4, 5, and 6, respectively.

In some embodiments, the first binding domain that specifically binds to CTLA4 in the CTLA4/TIGIT binding protein comprises a VH1 and a VL1, wherein the VH1 comprises an HCDR having 1, 2, 3, 4, or 5 amino acid mutations as compared to any one of the aforementioned HCDRs; and/or, the VL1 comprises an LCDR having 1, 2, 3, 4, or 5 amino acid mutations as compared to any one of the aforementioned LCDRs.

In some specific embodiments, the aforementioned amino acid mutation is an amino acid replacement, substitution, modification, deletion, and/or addition (such as conservative amino acid substitution), and the mutation does not affect or does not substantially affect the function of the first binding domain that specifically binds to CTLA4.

In some embodiments, in the first binding domain that specifically binds to CTLA4 in the CTLA4/TIGIT binding protein:

the VH1 comprises an amino acid sequence as set forth in SEQ ID NO: 13 or an amino acid sequence having at least 80%, at least 90% (as non-limiting examples, at least 90%, 91%, 92%, 93%, 94%, 95%, 96%, 97%, 98%, 99%, 100%) sequence identity thereto; and/or

the VL1 comprises an amino acid sequence as set forth in SEQ ID NO: 14 or an amino acid sequence having at least 80%, at least 90% (as non-limiting examples, at least 90%, 91%, 92%, 93%, 94%, 95%, 96%, 97%, 98%, 99%, 100%) sequence identity thereto.

In some embodiments, the first binding domain that specifically binds to CTLA4 in the CTLA4/TIGIT binding protein comprises an anti-CTLA4 antibody of tremelimumab, for example, comprising the Fab region or the Fv region of tremelimumab.

In some embodiments, the first binding domain that specifically binds to CTLA4 in the CTLA4/TIGIT binding protein comprises a heavy chain variable region (VH1) and a light chain variable region (VL1), wherein the VH1 comprises HCDR1, HCDR2, and HCDR3 as set forth in SEQ ID NOs: 27, 28, and 29, respectively, and the VL1 comprises LCDR1, LCDR2, and LCDR3 as set forth in SEQ ID NOs: 30, 31, and 32, respectively.

Regarding the second binding domain that specifically binds to TIGIT:

In some embodiments, the second binding domain that specifically binds to TIGIT in the CTLA4/TIGIT binding protein comprises a heavy chain variable region (VH2) and a light chain variable region (VL2), wherein the VH2 comprises HCDR1, HCDR2, and HCDR3 in an amino acid sequence as set forth in SEQ ID NO: 15, and the VL2 comprises LCDR1, LCDR2, and LCDR3 in an amino acid sequence as set forth in SEQ ID NO: 16.

The CDRs are defined according to the Kabat, IMGT, Chothia, AbM, or Contact numbering system. In some specific embodiments, the CDRs are defined according to the Kabat numbering system.

In some embodiments, the second binding domain that specifically binds to TIGIT in the CTLA4/TIGIT binding protein comprises a heavy chain variable region (VH2) and a light chain variable region (VL2), wherein the VH2 comprises HCDR1, HCDR2, and HCDR3 as set forth in SEQ ID NOs: 7, 8, and 9, respectively, and the VL2 comprises LCDR1, LCDR2, and LCDR3 as set forth in SEQ ID NOs: 10, 11, and 12, respectively.

In some embodiments, the second binding domain that specifically binds to TIGIT in the CTLA4/TIGIT binding protein comprises a VH2 and a VL2, wherein the VH2 comprises an HCDR having 1, 2, 3, 4, or 5 amino acid mutations as compared to any one of the aforementioned HCDRs; and/or, the VL2 comprises an LCDR having 1, 2, 3, 4, or 5 amino acid mutations as compared to any one of the aforementioned LCDRs.

In some specific embodiments, the aforementioned amino acid mutation is an amino acid replacement, substitution, modification, deletion, and/or addition (such as conservative amino acid substitution), and the mutation does not affect or does not substantially affect the function of the second binding domain that specifically binds to TIGIT.

In some embodiments, in the second binding domain that specifically binds to TIGIT in the CTLA4/TIGIT binding protein:

the VH2 comprises an amino acid sequence as set forth in SEQ ID NO: 15 or an amino acid sequence having at least 80%, at least 90% (as non-limiting examples, at least 90%, 91%, 92%, 93%, 94%, 95%, 96%, 97%, 98%, 99%, 100%) sequence identity thereto; and/or

the VL2 comprises an amino acid sequence as set forth in SEQ ID NO: 16 or an amino acid sequence having at least 80%, at least 90% (as non-limiting examples, at least 90%, 91%, 92%, 93%, 94%, 95%, 96%, 97%, 98%, 99%, 100%) sequence identity thereto.

In some embodiments, the second binding domain that specifically binds to TIGIT in the CTLA4/TIGIT binding protein comprises an anti-TIGIT antibody of tiragolumab, domvanalimab, vibostolimab, or ociperlimab, for example, comprising the Fab region or the Fv region of tiragolumab, domvanalimab, vibostolimab, or ociperlimab.

In some embodiments, the CTLA4/TIGIT binding protein further comprises a human immunoglobulin Fc region. In some specific embodiments, the Fc region is an Fc region of human IgG1, IgG2, IgG3, or IgG4, for example, an Fc region of human IgG1.

In some embodiments, the Fc region may allow the binding protein to form a dimeric molecule. In some embodiments, the Fc is conducive to extending the *in vivo* half-life of the binding protein.

In some embodiments, the Fc comprises an amino acid mutation at one or more positions selected from positions 239, 332, and 330 (IgG1, EU index numbering) (or at equivalent positions in other IgG isotypes). In some specific embodiments, the Fc has 239D/332E/330L, or 239D/332E mutations; exemplarily, S239D/I332E/A330L, or S239D/I332E.

In some embodiments, the Fc comprises mutations selected from

1) position 236; for example, 236A; exemplarily, G236A;

2) at least one of positions 298, 333, and 334; for example, 298A/333A/334A; exemplarily, S298A/E333A/K334A;

3) at least one of positions 235, 243, 292, 300, 305, and 396; for example, 243L/292P/300L/305I/396L, or 235V/243L/292P/300L/396L; exemplarily, F243L/R292P/Y300L/V305I/P396L, or L235V/F243L/R292P/Y300L/P396L;

4) 234Y/235Q/236W/239M/268D/270E/298A on one heavy chain and 270E/326D/330M/334E on the other heavy chain; exemplarily, L234Y/L235Q/ G236W/S239M/H268D/D270E/S298A on one heavy chain and D270E/K326-D/A330M/K334E on the other heavy chain.

In some embodiments, a mutation is introduced in the Fc region, wherein the mutation has the effect of enhancing the ADCC of the antibody.

In some embodiments, the Fc region comprises a first subunit (Fc1) and a second subunit (Fc2), and mutations are introduced that cause the two subunits (Fc1, Fc2) of the Fc region to pair to form a dimer, or mutations that reduce homodimerisation.

In some embodiments, the Fc region comprises knob-into-hole mutations that promote the association of the first subunit and the second subunit. For example, within the CH3/CH3 interface, one, two, or more amino acid residues in the CH3 domain of Fc1 are mutated with one or more amino acid residues having a larger side chain volume, thereby creating a protrusion (or Knob) on the surface of the CH3 domain of Fc1; and one, two, or more amino acid residues in the CH3 domain of Fc2 that interact with the CH3 domain of Fc1 are mutated with amino acid residues having a smaller side chain volume, thereby creating a cavity (or Hole) on the surface of the CH3 domain of Fc2 that interacts with the CH3 domain of Fc1.

In some embodiments, the Fc1 has one or more amino acid substitutions at positions selected from 354, 356, 358, and 366, and the Fc2 has one or more amino acid substitutions at positions selected from 349, 356, 358, 366, 368, and 407. In some specific embodiments, the Fc1 comprises a mutation at position 366, and the Fc2 comprises a mutation at a position selected from 366, 368, and 407, or any combination thereof. In some specific embodiments, the Fc1 comprises a mutation at position 354 or 356, and the Fc2 comprises a mutation at position 349. In some specific embodiments, the Fc1 comprises a mutation at position 354 or 356, and the Fc2 comprises mutations at positions 349, 366, 368, and 407.

In some embodiments, the Fc1 has one or more amino acid substitutions selected from 354C, 356E, 358M, and 366W, and the Fc2 has one or more amino acid substitutions selected from 349C, 356E, 358M, 366S, 368A, and 407V. In some specific embodiments, the Fc1 comprises a 366W mutation, and the Fc2 comprises a mutation selected from 366S, 368A, and 407V, or any combination thereof. In some specific embodiments, the Fc1 comprises a 354C or 356C mutation, and the Fc2 comprises a 349C mutation. Or in some specific embodiments, the Fc1 comprises 354C/366W mutations, and the Fc2 comprises 349C/366S/368A/407V mutations.

In some embodiments, the Fc1 has a sequence as set forth in SEQ ID: 25, and the Fc2 has a sequence as set forth in SEQ ID: 26.

In some embodiments, in the CTLA4/TIGIT binding protein, amino acid mutations are made to the size and charge of amino acids at the heavy chain CH1 and light chain CL interface, thereby reducing mispairing between the heavy chain and light chain. Exemplarily, Roche exchanged CH1 and CL domains and created a CrossMab platform (Schaefer et al. Proceedings of the National Academy of Sciences of the United States of America, 108 (27), pp. 11187-11192 (2011)); MedImmune introduced a disulphide bond through a F126C mutation on the heavy chain and a S121C mutation on the light chain (Mazor et al., mAbs, 7 (2), pp. 377-389 (2015)); Amgen further made electrostatic modifications in the CH1-CL region (Liu et al., Journal of Biological Chemistry, 290 (12), pp. 7535-7562 (2015)), and Lilly (Lewis et al., Nature Biotechnology, 32 (2), pp. 191-198 (2014)) and Genentech (Dillon et al., mAbs, 9 (2), pp. 213-230 (2017)) introduced mutations in both variable domain and constant domains. WuXi AppTec replaced the constant region of an antibody with the constant region of a TCR, as described in CN 109535257 A (incorporated herein by reference in its entirety).

In some embodiments, the CTLA4/TIGIT binding protein comprises a linker.

In some embodiments, the linker is an amino acid sequence as set forth in $(G_mS_n)_h$ or $(GGNGT)_h$ (SEQ ID NO: 44) or $(YGNGT)_h$ (SEQ ID NO: 45) or $(EPKSS)_h$ (SEQ ID NO: 46), wherein m and n are each independently selected from an integer of 1-8 (e.g., 1, 2, 3, 4, 5, 6, 7, or 8), h is independently selected from an integer of 1 to 20 (e.g., 1, 2, 3, 4, 5, 6, 7, 8, 9, 10, 11, 12, 13, 14, 15, 16, 17, 18, 19, or 20).

In some embodiments, the linker is a $(G_xS)_y$ linker, where x is selected from an integer of 1-5 (e.g., 1, 2, 3, 4, or 5), and y is selected from an integer of 1-6 (e.g., 1, 2, 3, 4, 5, or 6). In some embodiments, the linker is selected from $G_4S$ (SEQ ID NO: 47), GS, GAP, $(G_4S)_2$ (SEQ ID NO: 48), $(G_4S)_3$ (SEQ ID NO: 49), $(G_4S)_4$ (SEQ ID NO: 50), $(G_4S)_5$ (SEQ ID NO: 51), ASGS (SEQ ID NO: 52).

In some embodiments, the CTLA4/TIGIT binding protein comprises only one first binding domain that specifically binds to CTLA4 and at least one second binding domain that specifically binds to TIGIT.

In some specific embodiments, the CTLA4/TIGIT binding protein comprises only one first binding domain that specifically binds to CTLA4 and only one second binding domain that specifically binds to TIGIT.

In some specific embodiments, in the CTLA4/TIGIT binding protein, the first binding domain that specifically binds to CTLA4 is a Fab, and the second binding domain that specifically binds to TIGIT is a replaced Fab, wherein the replaced Fab comprises a Titin-T chain and an Obscurin-O chain, which are linked to the variable regions, that is, the original CH1 and CL of the Fab are replaced with the Titin-T chain and the Obscurin-O chain. In some specific embodiments, the VH2 of the second binding domain is fused, directly or via a linker, to the N-terminus of the Titin-T chain, and the VL2 of the second binding domain is fused, directly or via a linker, to the N-terminus of the Obscurin-O chain; or, the VH2 of the second binding domain is fused, directly or via a linker, to the N-terminus of the Obscurin-O chain, and the VL2 of the second binding domain is fused, directly or via a linker, to the N-terminus of the Titin-T chain.

In some specific embodiments, in the CTLA4/TIGIT binding protein, the second binding domain that specifically binds to TIGIT is a Fab, and the first binding domain that specifically binds to CTLA4 is a replaced Fab, wherein the replaced Fab comprises a Titin-T chain and an Obscurin-O chain, which are linked to the variable regions, that is, the original CH1 and CL of the Fab are replaced with the Titin-T chain and the Obscurin-O chain. In some specific embodiments, the VH1 of the first binding domain is fused, directly or via a linker, to the N-terminus of the Titin-T chain, and the VL1 of the first binding domain is fused, directly or via a linker, to the N-terminus of the Obscurin-O chain; or, the VH1 of the first binding domain is fused, directly or via a linker, to the N-terminus of the Obscurin-O chain, and the VL1 of the first binding domain is fused, directly or via a linker, to the N-terminus of the Titin-T chain.

The technique for the replacement of the Titin-T chain and Obscurin-O chain is described in detail in WO 2021139758 A1, which is incorporated herein by reference in its entirety.

In some embodiments, the CTLA4/TIGIT binding protein comprises a first heavy chain, a first light chain, a second heavy chain, and a second light chain; wherein

the first heavy chain, from the N-terminus to the C-terminus, sequentially comprises: [VH1]-[linker 1]-[Obscurin-O chain]-[linker 3]-[first subunit of Fc region],
the first light chain, from the N-terminus to the C-terminus, sequentially comprises: [VL1]-[linker 2]-[Titin-T chain],
the second heavy chain, from the N-terminus to the C-terminus, sequentially comprises: [VH2]-[CH1]-[second subunit of Fc region], and
the second light chain, from the N-terminus to the C-terminus, sequentially comprises: [VL2]-[CL]; or
the first heavy chain, from the N-terminus to the C-terminus, sequentially comprises: [VH2]-[linker 1]-[Obscurin-O chain]-[linker 3]-[first subunit of Fc region],
the first light chain, from the N-terminus to the C-terminus, sequentially comprises: [VL2]-[linker 2]-[Titin-T chain],
the second heavy chain, from the N-terminus to the C-terminus, sequentially comprises: [VH1]-[CH1]-[second subunit of Fc region], and
the second light chain, from the N-terminus to the C-terminus, sequentially comprises: [VL1]-[CL];
wherein

    -    represents a peptide bond, and the linker 1, linker 2, and linker 3 may be the same or different, and may independently be present or absent.

In some embodiments, the Obscurin-O chain has an amino acid sequence as set forth in SEQ ID NO: 17 or an amino acid sequence having at least 80% sequence identity thereto, and the Titin-T chain has an amino acid sequence as set forth in SEQ ID NO: 18 or an amino acid sequence having at least 80% sequence identity thereto.

In some specific embodiments, the linker 1 and the linker 2 are as set forth in GGGGS (SEQ ID NO: 35), and the linker 3 is absent.

In some embodiments, the CTLA4/TIGIT binding protein comprises the following combination of polypeptide chains:

a first heavy chain comprising an amino acid sequence as set forth in SEQ ID NO: 21 or an amino acid sequence having at least 90% sequence identity thereto,

a first light chain comprising an amino acid sequence as set forth in SEQ ID NO: 22 or an amino acid sequence having at least 90% sequence identity thereto,

a second heavy chain comprising an amino acid sequence as set forth in SEQ ID NO: 23 or an amino acid sequence having at least 90% sequence identity thereto, and/or

a second light chain comprising an amino acid sequence as set forth in SEQ ID NO: 24 or an amino acid sequence having at least 90% sequence identity thereto.

In some embodiments, provided is a CTLA4/TIGIT binding protein, which comprises a combination of polypeptide chains of SEQ ID NOs: 21-24.

In some embodiments, the CTLA4/TIGIT binding protein comprises two first binding domains that specifically bind to CTLA4 and two second binding domains that specifically bind to TIGIT.

In some embodiments, provided is a CTLA4/TIGIT binding protein, which comprises a combination of polypeptide chains of SEQ ID NOs: 33-35.

In some embodiments, the aforementioned CTLA4/TIGIT binding protein has at least one of the following characteristics:

a) specifically binding to human CTLA4 or an epitope thereof;

b) specifically binding to human TIGIT or an epitope thereof;

c) having binding activity to cells co-expressing CTLA4 and TIGIT that is superior to binding activity to CTLA4 single-positive cells; for example, an $EC_{50}$ ratio of the two is $\geq 2$; exemplarily, the $EC_{50}$ ratio is $\geq 2$, the $EC_{50}$ ratio is $\geq 3$, the $EC_{50}$ ratio is $\geq 4$, the $EC_{50}$ ratio is $\geq 5$, the $EC_{50}$ ratio is $\geq 6$, the $EC_{50}$ ratio is $\geq 7$, the $EC_{50}$ ratio is $\geq 8$, the $EC_{50}$ ratio is $\geq 9$, the $EC_{50}$ ratio is $\geq 10$, the $EC_{50}$ ratio is $\geq 20$, and the $EC_{50}$ ratio is $\geq 30$ or higher;

d) having blocking activity against CTLA4-CD80 in the presence of both CTLA4 and TIGIT that is superior to blocking activity against CTLA4-CD80 in the presence of CTLA4 only; for example, an $I_{50}$ ratio of the two is $\geq 2$, the $IC_{50}$ ratio is $\geq 3$, the $IC_{50}$ ratio is $\geq 4$, the $I_{50}$ ratio is $\geq 5$, the $IC_{50}$ ratio is $\geq 6$, the $IC_{50}$ ratio is $\geq 7$, the $IC_{50}$ ratio is $\geq 8$, the $IC_{50}$ ratio is $\geq 9$, the $I_{50}$ ratio is $\geq 10$, the $IC_{50}$ ratio is $\geq 20$, and the $I_{50}$ ratio is $\geq 30$ or higher;

e) depleting or inhibiting Treg cells, e.g., intratumoural Treg cells;

f) activating T cells and/or promoting T cell proliferation; for example, activating intratumoural CD8+T cells;

g) inhibiting tumour growth, or treating or alleviating cancer.

In some embodiments, the aforementioned CTLA4/TIGIT binding protein of the present disclosure is capable of inhibiting tumour growth by at least about 10%, such as by at least about 20%, about 30%, about 40%, about 50%, about 60%, about 70%, about 80%, or about 90%.

In some embodiments, the CTLA4/TIGIT binding protein of the present disclosure is an anti-CTLA4/TIGIT antibody, such as an anti-CTLA4/TIGIT bispecific antibody or an anti-CTLA4/TIGIT multispecific antibody.

The antibody includes an antigen-binding fragment, which includes but is not limited to, Fab, Fv, sFv, Fab', F(ab')2, a linear antibody, a single-chain antibody, scFv, sdAb, sdFv, a nanobody, a peptibody, a domain antibody, a diabody, a triabody, a tetrabody, tandem di-scFv, and tandem tri-scFv. In some specific embodiments, the antigen-binding fragment includes Fab, Fv, sFv, Fab', and F(ab')$_2$.

In some embodiments, provided is an anti-CTLA4/TIGIT antibody, which competes with the aforementioned CTLA4/TIGIT binding protein of the present disclosure for binding to CTLA4 and/or TIGIT, or for binding to the same epitope on CTLA4 and/or TIGIT.

In some embodiments, provided is an anti-CTLA4/TIGIT antibody, which blocks the binding of the aforementioned CTLA4/TIGIT binding protein of the present disclosure to CTLA4 and/or TIGIT.

In some embodiments, provided is a protein or molecule, which comprises any one of the aforementioned CTLA4/TIGIT binding proteins of the present disclosure. For example, the protein or molecule is a conjugate, which may, for example, comprises any detectable label.

The CTLA4/TIGIT binding protein (for example, the anti-CTLA4/TIGIT antibody) provided by the present disclosure can, via an avidity effect, increase the binding capacity to cells co-expressing CTLA4 and TIGIT and enhance the blocking activity against CTLA4-CD80.

The CTLA4/TIGIT binding protein provided by the present disclosure selectively depletes intratumoural Treg cells, leads to an increase in intratumoural CD8+T cells, reduces toxicity, improves anti-tumour activity, has a favourable administration window and outstanding potential to be manufactured as a drug, and provides a solution for the clinical treatment of tumours.

Polynucleotide and vector

The present disclosure provides a polynucleotide, which encodes the CTLA4/TIGIT binding protein provided by the present disclosure.

In some embodiments, the polynucleotide may be RNA, DNA, or cDNA. According to some embodiments of the present disclosure, the polynucleotide of the present disclosure is a substantially isolated nucleic acid.

The nucleic acid of the present disclosure may also be in the form of a vector, may be present in a vector, and/or may be part of a vector, such as a plasmid, a cosmid, YAC, or a viral vector. The vector may in particular be an expression vector, i.e., a vector that enables the expression of the CTLA4/TIGIT binding protein *in vitro* and/or *in vivo* (i.e., in a suitable host cell, host organism, and/or expression system). The expression vector generally comprises at least one nucleic acid of the present disclosure operably linked to one or more suitable expression regulatory elements (e.g., promoters, enhancers, and terminators). It is common knowledge to those skilled in the art to select the elements and their sequences for expression in a particular host. Regulatory elements and other elements useful or necessary for the expression of the CTLA4/TIGIT binding protein of the present disclosure include, for example, promoters, enhancers, terminators, integration factors, selectable markers, leader sequences, and reporter genes.

The nucleic acid of the present disclosure may be prepared or obtained by known methods (e.g., by automatic DNA synthesis and/or recombinant DNA technology) based on the information of the amino acid sequence of the polypeptide of the present disclosure, and/or may be isolated from a suitable natural source.

Host cell

The present disclosure provides a recombinant host cell that expresses or is capable of expressing one or more CTLA4/TIGIT binding proteins of the present disclosure and/or comprises the nucleic acid or vector of the present disclosure.

In some embodiments, the host cell is a bacterial cell, a fungal cell, or a mammalian cell.

Exemplarily, bacterial cells include, for example, cells from Gram-negative bacterial strains (e.g., *Escherichia coli* strains, *Proteus* strains, and *Pseudomonas* strains) and Gram-positive bacterial strains (e.g., *Bacillus* strains, *Streptomyces* strains, *Staphylococcus* strains, and *Lactococcus* strains).

Exemplarily, fungal cells include, for example, cells of species of *Trichoderma, Neurospora,* and *Aspergillus;* or cells of species of *Saccharomyces* (e.g., *Saccharomyces cerevisiae*)*, Schizosaccharomyces* (e.g.*, Schizosaccharomyces pombe), Pichia* (e.g., *Pichia pastoris* and *Pichia methanolica*)*, and *Hansenula.*

Exemplarily, mammalian cells include, for example, HEK293 cells, CHO cells, BHK cells, HeLa cells, and COS cells.

Amphibian cells, insect cells, plant cells, and any other cells used in the art to express heterologous proteins may also be used in the present disclosure.

The cells of the present disclosure are not capable of developing into a complete plant or an entire animal organism.

Production or preparation method

The present disclosure provides a method for preparing any of the CTLA4/TIGIT binding protein of the present disclosure. In some embodiments, the method comprises the following steps:

-    culturing the host cell of the present disclosure under conditions suitable for expression of the antibody; and
-    recovering a protein of interest expressed by the host cell from the culture; and
-    optionally, the method comprises: further purifying and/or modifying the protein of interest of the present disclosure.

The CTLA4/TIGIT binding protein of the present disclosure may be produced intracellularly (e.g., in the cytoplasm, in the periplasm, or in inclusion bodies) in the cells described above, followed by isolation from the host cell and optionally, further purification; or the CTLA4/TIGIT binding protein may be produced extracellularly (e.g., in the culture medium where the host cell is cultured), followed by isolation from the culture medium and optionally, further purification.

Methods and reagents for producing polypeptides by recombination, such as specifically suitable expression vectors, transformation or transfection methods, selectable markers, methods for inducing protein expression, and culture conditions, are known in the art. Similarly, isolation and purification techniques suitable for manufacturing a protein of interest such as the binding protein or antibody of the present disclosure are well known to those skilled in the art. Methods for producing and purifying antibodies are well known and available in the prior art, for example, in Antibodies: A Laboratory Manual, Cold Spring Harbor Laboratory Press (Chapters 5-8 and 15). The engineered antibodies of the present disclosure may also be prepared and purified by conventional methods. For example, cDNA sequences encoding the heavy chain and light chain may be cloned and recombined into an expression vector. Recombinant immunoglobulin expression vectors can be stably transfected into cells. Mammalian expression systems result in glycosylation of antibodies, especially at the highly conserved N-terminus of the Fc region. Stable clones are obtained by expressing antibodies that specifically bind to human antigens. Positive clones are expanded in a serum-free medium in a bioreactor to produce antibodies. The culture medium containing the secreted antibodies may be purified and collected by conventional techniques. The antibodies may be filtered and concentrated by conventional methods. Soluble aggregates and multimers may also be removed by conventional methods, such as molecular sieving and ion exchange. The resulting product should be immediately frozen (e.g., at -70°C) or lyophilised.

However, the CTLA4/TIGIT binding protein of the present disclosure may also be obtained by other methods for producing proteins known in the art, such as chemical synthesis, including solid-phase or liquid-phase synthesis.

Composition

The present disclosure provides a composition comprising any one or any combination of the following: any of the CTLA4/TIGIT binding protein, the polynucleotide encoding the CTLA4/TIGIT binding protein, and the vector, provided by the present disclosure.

In some embodiments, the pharmaceutical composition comprises the aforementioned CTLA4/TIGIT binding protein, polynucleotide encoding the CTLA4/TIGIT binding protein, or vector in an amount effective to treat, alleviate, or prevent cancer.

In some embodiments, the pharmaceutical composition further comprises at least one pharmaceutically acceptable excipient, diluent, or carrier.

In some specific embodiments, the pharmaceutical composition may contain 0.01% to 99% by weight of the CTLA4/TIGIT binding protein in a unit dose; the amount of the CTLA4/TIGIT binding protein in a unit dose of the pharmaceutical composition is 0.1-2000 mg, and in some specific embodiments, 1-1000 mg.

In some embodiments, provided is an article of manufacture or product, which comprises the aforementioned CTLA4/TIGIT binding protein, polynucleotide, and/or vector. Optionally, the article of manufacture comprises a container and a label. The container is, for example, a vial, a syringe, or a test tube. The container contains a composition effective for treating a condition. The label on or attached to the container indicates that the composition is used to treat the selected condition. The composition comprises the aforementioned CTLA4/TIGIT binding protein, polynucleotide, and/or vector.

Kit and detection

The present disclosure provides a kit comprising the aforementioned CTLA4/TIGIT binding protein, polynucleotide, vector, or composition. The present disclosure further provides a method, system, or device for detecting CTLA4 and TIGIT *in vivo* or *in vitro,* comprising: treating a sample using the aforementioned binding protein, polynucleotide, or composition of the present disclosure.

In some embodiments, the *in vitro* detection method, system, or device may, for example, comprise:

(1) contacting a sample with the CTLA4/TIGIT binding protein, polynucleotide, vector, or composition of the present disclosure;
(2) detecting a complex formed between the aforementioned binding protein, polynucleotide, vector, or composition and the sample; and/or
(3) contacting a reference sample (e.g., a control sample) with the binding protein or polynucleotide; and
(4) determining the extent of complex formation by comparison with the reference sample. A change in complex formation in the sample or a subject (e.g., a statistically significant change) as compared to a control sample or subject indicates the presence of CTLA4 and TIGIT in the sample.

In other embodiments, the *in vivo* detection method, system, or device may comprise:

(1) administering the aforementioned binding protein, polynucleotide, or vector to a subject; and
(2) detecting the formation of a complex between the aforementioned binding protein, polynucleotide, or vector and the subject.

Detection may comprise determining the location or time of complex formation. The aforementioned binding protein or nucleic acid is labelled with a detectable substance, and detection of the label enables the detection of a substance (e.g., CTLA4, TIGIT) that can bind to the protein or nucleic acid. Suitable detectable substances include a variety of enzymes, prosthetic groups, fluorescent substances, luminescent substances, and radioactive substances. Complex formation between the binding protein or nucleic acid and CTLA4 or TIGIT may be detected by measuring or visualising a substance that binds or does not bind to CTLA4 or TIGIT. Conventional detection assays may be used, for example, enzyme-linked immunosorbent assay (ELISA), radioimmunoassay (RIA), or tissue immunohistochemistry. For detection purposes, the binding protein or nucleic acid of the present disclosure may be labelled with a fluorophore chromophore. In some embodiments, further provided are a diagnostic reagent comprising the aforementioned nucleic acid or binding protein, and related diagnostic uses.

In some embodiments, further provided is a kit, which comprises the aforementioned binding protein or polynucleotide, and may further comprise instructions for diagnostic use. The kit may also comprise at least one additional reagent, such as a marker or an additional diagnostic agent. For *in vivo* use, the binding protein may be formulated as a pharmaceutical composition.

Method of treating diseases and pharmaceutical use

Provided are the use of, and method using, the CTLA4/TIGIT binding protein, the polynucleotide encoding the CTLA4/TIGIT binding protein, the vector, or the pharmaceutical composition, provided by the present disclosure, in the prevention, treatment, or alleviation of diseases or conditions.

In some embodiments, provided is at least one of the following uses of the CTLA4/TIGIT binding protein, the polynucleotide encoding the CTLA4/TIGIT binding protein, the vector, or the pharmaceutical composition, provided by the present disclosure:

1) for use in the manufacture of a medicament for preventing, treating, or alleviating diseases or conditions;
2) for use in preventing, treating, or alleviating diseases or conditions;
3) for use in activating T cells;
4) for use in the manufacture of a medicament for activating T cells;
5) for use in depleting or inhibiting Treg cells;
6) for use in the manufacture of a medicament for depleting or inhibiting Treg cells;
7) for use in selectively killing CTLA4 and TIGIT double-positive cells;
8) for use in the manufacture of a medicament for selectively killing CTLA4 and TIGIT double-positive cells;
9) for use in reducing peripheral toxicity, ameliorating toxic and side effects, and improving safety.

In some embodiments, the present disclosure provides a method for preventing, treating, or alleviating diseases or conditions, which method comprises administering to a patient or subject the CTLA4/TIGIT binding protein, polynucleotide encoding the CTLA4/TIGIT binding protein, vector, or pharmaceutical composition of the present disclosure in an amount effective to prevent, treat or alleviate diseases or conditions.

In some embodiments, the diseases or conditions are tumours or cancer. In some specific embodiments, the cancer is colourectal cancer, non-small cell lung cancer, or breast cancer.

In some embodiments, the CTLA4/TIGIT binding protein, polynucleotide encoding the CTLA4/TIGIT binding protein, vector, or pharmaceutical composition of the present disclosure may be administered, either systemically or topically, by any suitable method known in the art.

In some embodiments, the administration regimen may be adjusted to achieve the optimal desired response (e.g., a therapeutic or prophylactic response). For example, a single dose may be administered, multiple doses may be administered over a period of time, or the dose may be proportionally reduced or increased depending on the urgency of the treatment situation.

The ordinal numerals "first", "second", "third", "1", "2", "3", etc. (e.g., "first subunit", "Fc2", "third chain", "linker 2") in the present disclosure are used solely to distinguish different features, elements, components, or steps, and are not intended to limit quantity, order, or hierarchy.

**Brief Description of the Drawings**

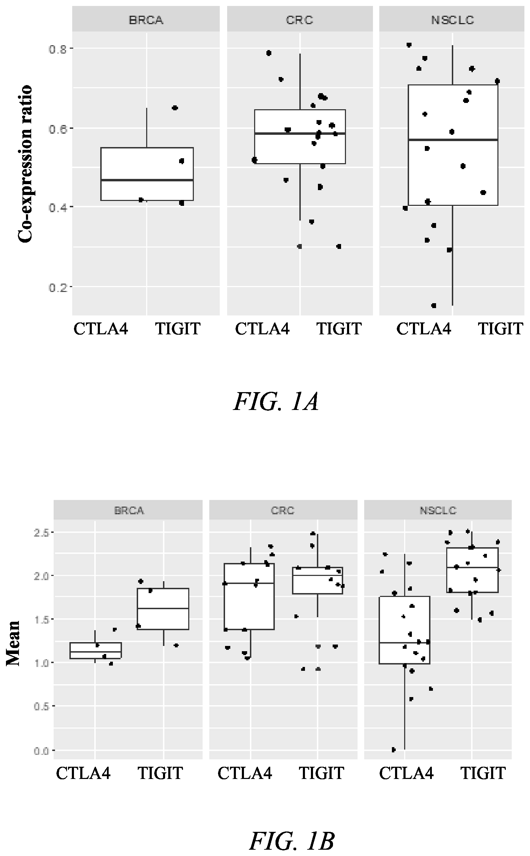

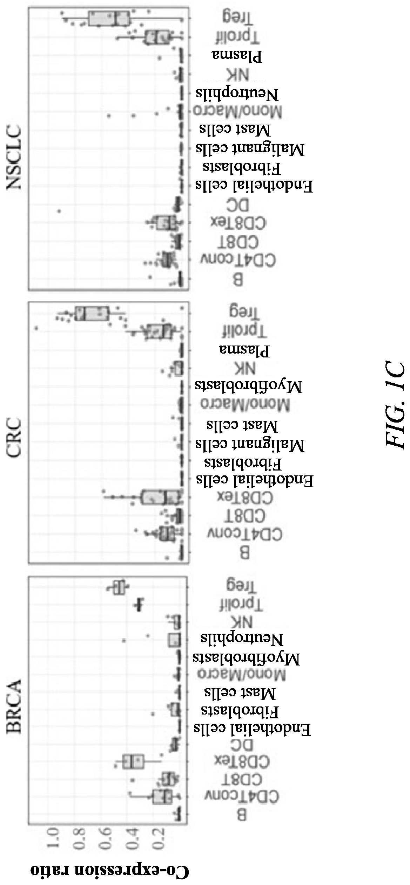

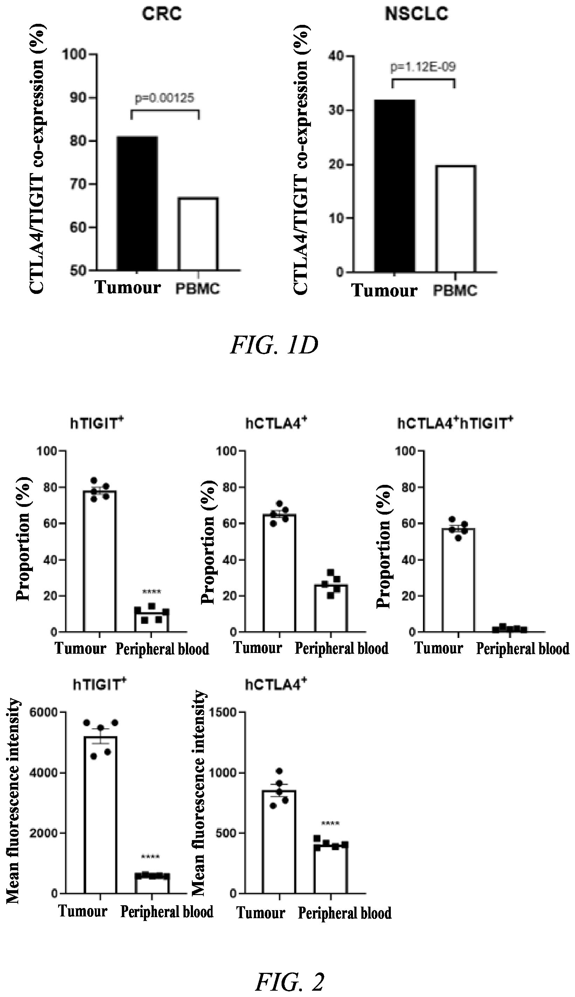

FIG. 1A to FIG. 1D show the expressions of CTLA4 and TIGIT in tumour patient samples on the basis of analysis of single-cell sequencing results using bioinformatics, where FIG. 1A shows the co-expression ratio of CTLA4 and TIGIT on intratumoural Tregs across breast cancer (BRCA), colourectal cancer (CRC), and non-small cell lung cancer (NSCLC); FIG. 1B shows the expression levels of CTLA4 and TIGIT on intratumoural Tregs across BRCA, CRC, and NSCLC; FIG. 1C shows the co-expression ratio of CTLA4 and TIGIT across various immune cells; and FIG. 1D shows the co-expression ratio of CTLA4 and TIGIT on intratumoural Tregs of CRC or NSCLC patients.

FIG. 2 shows the analysis of the CTLA4 expression level, TIGIT expression level, or CTLA4/TIGIT co-expression level on intratumoural Tregs, as detected by flow cytometry, in CTLA4-TIGIT double-humanised mouse MC38 tumour xenograft model.

FIG. 3A-FIG. 3C show the binding activity of anti-CTLA4/TIGIT antibody to CTLA4 and TIGIT antigens on the cell surface, as detected by flow cytometry, where FIG. 3A and FIG. 3B show the binding to CHOK1-CTLA4 cells, and FIG. 3C shows the binding to CHOK1-TIGIT cells.

FIG. 4A and FIG. 4B show the blocking activity of anti-CTLA4/TIGIT antibody against CTLA4-CD80 and TIGIT-CD155, as detected by flow cytometry, where FIG. 4A shows blocking of the CTLA4-CD80 binding, and FIG. 4B shows blocking of TIGIT-CD155 binding.

FIG. 5A and FIG. 5B show the binding activity of anti-CTLA4/TIGIT antibody to HEK293-CTLA4 single-positive cells and HEK293-CTLA4/TIGIT double-positive cells, as detected by flow cytometry, where FIG. 5A shows the binding activity of Ipi, and FIG. 5B shows the binding activity of As-3.

FIG. 6 shows the blocking activity of anti-CTLA4/TIGIT antibody against CTLA4-CD80, as detected by ELISA.

FIG. 7A and FIG. 7B show the anti-CTLA4/TIGIT antibody-mediated ADCC killing activity of NK against HEK293-CTLA4 single-positive cells and HEK293-CTLA4/TIGIT double-positive cells, as detected by LDH assay, where FIG. 7A shows the ADCC killing activity of Ipi, and FIG. 7B shows the ADCC binding activity of As-3.

FIG. 8 shows the anti-CTLA4/TIGIT antibody-mediated ADCC killing activity of NK against iTreg, as detected by LDH assay.

FIG. 9 shows the anti-CTLA4/TIGIT antibody-mediated CDC killing activity, as detected by CTG detection.

FIG. 10A to FIG. 10B show the inhibitory effect of anti-CTLA4/TIGIT antibody on MC38 tumour xenografts in CTLA4-TIGIT double-humanised mice.

FIG. 11 shows the effect of anti-CTLA4/TIGIT antibody on intratumoural and peripheral Tregs and CD8+ T cells in CTLA4-TIGIT double-humanised mouse MC38 tumour xenograft models.

FIG. 12A and FIG. 12B show the exemplary structures of the anti-CTLA4/TIGIT antibody.

## Detailed Description of Embodiments

Term definition

In order that the present disclosure can be more readily understood, certain technical and scientific terms are specifically defined below. Unless expressly defined otherwise in the present disclosure, all other technical and scientific terms used in the present disclosure have the meanings commonly understood by one of ordinary skill in the art to which the present disclosure belongs.

The three-letter and single-letter codes for amino acids used in the present disclosure are described in J. biol. chem, 243, p 3558 (1968).

"CTLA4" or "CTLA4" or "CTLA4 protein", or "CTLA4 polypeptide" can optionally include any such proteins or variants, conjugates, or fragments thereof, including (but not limited to) known or wild-type CTLA4 as described herein, and any naturally occurring splice variants, amino acid variants, or isoforms. The complete CTLA4 sequence can be found under GenBank accession number NP_005205.

"TIGIT" or "TIGIT protein", or "TIGIT polypeptide" can optionally include any such proteins or variants, conjugates, or fragments thereof, including (but not limited to) known or wild-type TIGIT as described herein, and any naturally occurring splice variants, amino acid variants, or isoforms. The complete TIGIT sequence can be found under GenBank accession number NP_776160.

The term "functional variant" includes, but is not limited to, homologs, fragments, truncations, mutants, modifications, etc. of a wild-type protein. Compared with the wild-type protein, a functional variant of a protein exhibits increased, decreased, or maintained protein activity.

The "binding protein" or "binding molecule" of the present disclosure encompasses any protein, polypeptide, or any molecule comprising the protein or polypeptide that is capable of specifically binding to an antigen (e.g., CTLA4 or TIGIT) or a fragment thereof or an epitope thereof, including but not limited to the antibodies as defined in the present disclosure.

In the present disclosure, the "polypeptide", "peptide", and "protein" are used interchangeably and refer to a polymer of amino acid residues, or a collection of polymers of multiple amino acid residues. These terms apply to amino acid polymers in which one or more amino acid residues are artificial chemical mimics of corresponding naturally occurring amino acids, as well as to both naturally occurring amino acid polymers and non-naturally occurring amino acid polymers. Polypeptide sequences are generally described with the left-hand end of the polypeptide sequence denoting the amino-terminus (N-terminus, N-end), and the right-hand end of the polypeptide sequence denoting the carboxyl-terminus (C-terminus, C-end).

"Antibody" encompasses a variety of antibody structures, including, but not limited to, monoclonal antibodies, polyclonal antibodies, monospecific antibodies, bispecific or multispecific antibodies (e.g., trispecific antibodies), full-length antibodies, and antibody fragments (or antigen-binding fragments, antigen-binding domains), so long as they exhibit the desired antigen-binding activity. The antibodies of the present disclosure include a recombinant antibody form.

An antibody may refer to an immunoglobulin, which is a tetrapeptide chain structure composed of two heavy chains and two light chains linked via interchain disulphide bonds. The immunoglobulin heavy chain constant regions have different amino acid compositions and sequence arrangements, and therefore have different antigenicity. Accordingly, immunoglobulins can be classified into five classes, or referred to as the isotypes of immunoglobulins, namely IgM, IgD, IgG, IgA, and IgE, whose corresponding heavy chains are the $\mu$ chain, $\delta$ chain, $\gamma$ chain, $\alpha$ chain, and $\varepsilon$ chain, respectively. Ig of the same class can be further classified into different subclasses based on differences in the amino acid composition of the hinge regions and the number and positions of the heavy chain disulphide bonds, for example, IgG can be divided into IgG1, IgG2, IgG3, and IgG4. Light chains are classified into $\kappa$ chains or $\lambda$ chains based on differences in the constant regions. Each of the five classes of Ig can have $\kappa$ chains or $\lambda$ chains. In the antibody heavy chains and light chains, the sequences of about 110 amino acids near the N-termini vary greatly, and are referred to as the variable regions (V regions); and the remaining amino acid sequences near the C-termini are relatively stable, and are referred to as the constant regions (C regions). The variable region comprises three hypervariable regions (HVRs) and four framework regions (FRs) with relatively conserved sequences. The three hypervariable regions determine the specificity of the antibody, and are also referred to as complementarity determining regions (CDRs). Each light chain variable region (VL) and heavy chain variable region (VH) consists of 3 CDR regions and 4 FR regions, arranged in the following order from the amino-terminus to the carboxyl-terminus: FR1, CDR1, FR2, CDR2, FR3, CDR3, FR4. The three CDR regions of the light chain refer to LCDR1, LCDR2, and LCDR3; the three CDR regions of the heavy chain refer to HCDR1, HCDR2, and HCDR3.

The "antigen-binding fragment" or "antigen-binding domain" encompasses Fab, modified Fab, Fab', modified Fab', F(ab')2, Fv, Fab-Fv, Fab-dsFv, single-domain antibodies (e.g., VH, or VL, or VHH), scFv, bivalent or trivalent or tetravalent antibodies, Bis-scFv, diabody, tribody, triabody, tetrabody, and epitope-binding fragments of any one of the foregoing (see, e.g., Holliger and Hudson, 2005, Nature Biotech. 23 (9): 1126-1136; Adair and Lawson, 2005, Drug Design Reviews-Online 2 (3), 209-217). Methods for producing and preparing these antigen-binding fragments are well-known in the art (see, e.g., Verma et al., 1998, Journal of Immunological Methods, 216, 165-181).

For the determination or definition of CDRs, definitive delineation of CDRs and identification of residues comprising the binding site of an antibody can be accomplished by resolving the structure of the antibody and/or resolving the structure of the antibody-ligand complex. This can be achieved by any of a variety of techniques known to those skilled in the art, such as X-ray crystallography. A variety of analytical methods can be used to identify CDRs, including but not limited to the Kabat numbering system, the Chothia numbering system, the AbM numbering system, the IMGT numbering system, contact definition, and conformational definition.

The Kabat numbering system is a standard for numbering residues in antibodies, and is commonly used to identify CDR regions (see, e.g., Johnson & Wu, 2000, Nucleic Acids Res., 28: 214-8). The Chothia numbering system is similar to the Kabat numbering system, but the Chothia numbering system contemplates the positions of certain structural loop regions. (See, e.g., Chothia et al., 1986, J. Mol. Biol., 196: 901-17; Chothia et al., 1989, Nature, 342: 877-83). The AbM numbering system uses an integrated suite of computer programs produced by Oxford Molecular Group that models antibody structures (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd). The AbM numbering system uses a combination of knowledge databases and *de novo* methods to model the tertiary structure of antibodies from the primary sequence (see those described by Samudrala et al., 1999, in "Ab Initio Protein Structure Prediction Using a Combined Hierarchical Approach" in PROTEINS, Structure, Function and Genetics Suppl., 3: 194-198). The contact definition is based on the analysis of available complex crystal structures (see, e.g., MacCallum et al., 1996, J. Mol. Biol., 5: 732-45). In the conformational definition, the positions of CDRs can be identified as residues that make enthalpic contributions to antigen binding (see, e.g., Makabe et al., 2008, Journal of Biological Chemistry, 283: 1156-1166). Additionally, other CDR boundary definitions may not strictly adhere to one of the above-mentioned methods but still overlap with at least a portion of the Kabat CDRs, even though they may be shortened or lengthened based on the fact that specific residues or groups of residues do not significantly affect antigen binding (as confirmed by predictive or experimental results). As used in the present disclosure, a CDR may refer to a CDR defined by any method known in the art, including combinations of such methods. The correspondences among various numbering systems are well-known to those skilled in the art, exemplary, as shown in Table 1 below.

EP 4 772 539 A1

Table 1. Relationships among CDR numbering systems

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues in the VL and VH regions of the antibodies of the present disclosure conform in both number and position to the known Kabat numbering system.

Although in specific embodiments, a numbering system (such as Kabat) is used to define amino acid residues, the corresponding technical solutions defined by other numbering systems shall be regarded as equivalent technical solutions.

"Binding affinity" or "affinity" is used in the present disclosure as a measure of the strength of the non-covalent interaction between two molecules (e.g., one antigen-binding site of an antibody and a corresponding antigenic epitope). The binding affinity between the two molecules can be quantified by determining the equilibrium dissociation constant ($K_D$). $K_D$ can be determined by measuring the kinetics of complex formation and dissociation by using, for example, the surface plasmon resonance (SPR) method (Biacore). The rate constants corresponding to the association and dissociation of a complex are referred to as the association rate constant ka (or kon) and the dissociation rate constant kd (or koff), respectively. $K_D$ is related to ka and kd by the equation $K_D$ = kd/ka. The value of the dissociation constant can be directly determined by well-known methods, and can even be calculated for complex mixtures by, for example, those methods described in Caceci et al. (1984, Byte 9: 340-362). For example, $K_D$ can be determined by using a dual filtration nitrocellulose filter binding assay such as that disclosed in Wong & Lohman (1993, Proc. Natl. Acad. Sci. USA 90: 5428-5432). Other standard assays for evaluating the binding capacity of an antibody to a target antigen are known in the art, and include, for example, ELISA, Western blotting, RIA, and FACS, as well as other assays exemplified elsewhere in the present disclosure. The binding kinetics and binding affinity of an antibody can also be evaluated by standard assays known in the art, such as surface plasmon resonance (SPR), for example, by using the Biacore™ system or KinExA. The binding affinities associated with different molecular interactions, e.g., the binding affinities of different antibodies for a given antigen, can be compared by comparing the $K_D$ values of various antibody/antigen complexes. Similarly, the specificity of an interaction can be evaluated by determining and comparing the $K_D$ value for the interaction of interest (e.g., the specific interaction between an antibody and an antigen) with the $K_D$ value for an interaction not of interest.

Avidity refers to the binding strength between the antigen-binding sites of an intact antibody molecule and several corresponding antigenic epitopes. Exemplarily, an anti-CTLA4/TIGIT antibody binds to CTLA4 and TIGIT antigens.

Generally, "specific binding" refers to the binding of a binding molecule (binding protein) to an epitope on an antigen. The CTLA4/TIGIT binding protein of the present disclosure may bind to the antigen to be bound (i.e., CTLA4 or TIGIT) or an epitope thereof with an equilibrium dissociation constant ($K_D$) of $\leq 10^{-7}$M, preferably $\leq 10^{-8}$M, as measured in assays such as Biacore, KinExA, or Fortibio. Any $K_D$ value greater than $10^{-4}$ M is generally considered to indicate non-specific binding. The specific binding of a binding molecule to an antigen or epitope may be determined in any known suitable manner, including, for example, surface plasmon resonance (SPR), enzyme-linked immunosorbent assay (ELISA), and/or fluorescence-activated cell sorting (FACS), as described in the present disclosure.

"Conservative substitution" refers to a substitution with another amino acid residue having similar properties to the original amino acid residue. For example, lysine, arginine, and histidine have similar properties in that they have basic side chains, and aspartic acid and glutamic acid have similar properties in that they have acidic side chains. Furthermore, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is substituted, it will not exhibit a particular change in properties.

"Homology", "identity", or "sequence identity" refers to sequence similarity between two nucleotide sequences or between two polypeptides. When positions in both of the two compared sequences are occupied by identical nucleotides or amino acid monomers, e.g., if a position of each of two DNA molecules is occupied by an identical nucleotide, the molecules are homologous at that position. The percent homology between two sequences is a function of the number of matched or homologous positions shared by the two sequences divided by the number of positions compared × 100%. For example, if 6 out of 10 positions are matched or homologous in two sequences when the sequences are optimally aligned, the two sequences are 60% homologous. Generally, a comparison is made when two sequences are aligned to give maximum percent homology.

The "Obscurin-O chain" or "O chain" refers to a peptide segment of the Obscurin protein, 87-117 amino acids in length, comprising the Obscurin Ig-like 1 domain, or a functional variant thereof. The Obscurin-O chain is capable of forming a complex with the Titin Ig-like 152 domain through intermolecular interactions. The functional variant of the Obscurin-O chain is a peptide where some of the amino acids of the wild-type O chain are mutated, but which still possesses the ability to form a complex with the Titin Ig-like 152 domain through intermolecular interactions.

The "Titin-T chain" or "T chain" refers to a peptide segment of the Titin protein, 78-118 amino acids in length, comprising the Titin Ig-like 152 domain, or a functional variant thereof. The Titin-T chain is capable of binding to the Obscurin Ig-like 1 or Obscurin Ig-like 1 domain to form a complex through intermolecular interactions. The functional variant of the T chain is a peptide where some of the amino acids of the wild-type T chain are mutated, but which still possesses the ability to bind to the Obscurin Ig-like 1 or Obscurin Ig-like 1 to form a complex through intermolecular interactions.

The Obscurin-O chain or Titin-T chain may be used to replace the CH1 or CL domain of an antibody without affecting the binding of the VH and VL of the antibody and the binding of the antibody to an antigenic epitope.

"Nucleic acid" and "polynucleotide" are used interchangeably herein to refer to any DNA or RNA molecule, either single- or double-stranded, and in the case of a single-stranded molecule, a molecule of its complementary sequence, preferably a double-stranded DNA.

The term "pharmaceutically acceptable adjuvant" or "pharmaceutically acceptable excipient" includes any material that, when combined with an active ingredient, allows the ingredient to retain biological activity and does not react with the immune system of the subject. Examples include, but are not limited to, any standard pharmaceutical carriers such as buffered saline solution, water, emulsions such as oil/water emulsions, and various types of wetting agents.

"Inhibition" and "blockade" are used interchangeably, and encompass both partial and complete inhibition/-blockade. "Inhibition of growth" (e.g., involving cells) is intended to include any measurable reduction in cell growth.

"Proliferative disease" refers to a condition associated with some degree of abnormal cell proliferation. In one embodiment, the proliferative condition refers to cancer. The "tumour" refers to all neoplastic cell growth and proliferation, whether malignant or benign, and all pre-cancerous and cancerous cells and tissues. The "cancer", "cancerous", "proliferative condition", and "tumour" are not mutually exclusive when referred to in the present disclosure.

"Giving", "administering", and "treating", when applied to animals, humans, experimental subjects, cells, tissues, organs, or biological fluids, refer to contact of an exogenous drug, therapeutic agent, diagnostic agent, or composition with the animals, humans, subjects, cells, tissues, organs, or biological fluids, for example in therapeutic, pharmacokinetic, diagnostic, research, and experimental methods. Treatment of cells includes contacting reagent with cells and contacting reagents with fluids, wherein the fluid is in contact with the cells. "Giving", "administering", and "treating" also means treating, e.g., cells, with a reagent, a diagnostic agent, a binding composition, or with another cell *in vitro* and *ex vivo.* When applied to humans, veterinary, or research subjects, they refer to therapeutic treatment, preventive or prophylactic measures, and research and diagnostic applications.

"Treating" means administering a therapeutic agent, such as a therapeutic agent comprising any binding protein or pharmaceutical composition thereof of the present disclosure, either internally or externally, to a subject who has had, is suspected of having, or is predisposed to having one or more proliferative diseases or symptoms thereof, on which the therapeutic agent is known to have a therapeutic effect. Typically, the therapeutic agent is administered in an amount effective to alleviate one or more disease symptoms in the subject or population being treated, whether by inducing regression of such symptoms or inhibiting the development of such symptoms to any clinically measurable degree. The amount of therapeutic agent effective to alleviate any particular disease symptom (also referred to as the "therapeutically effective amount") may vary depending on various factors such as the disease state, age, and weight of the subject, and the ability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating disease symptoms of interest in a certain subject, they shall alleviate the disease symptoms of interest in a statistically significant number of subjects, as determined by any statistical test method known in the art, such as the Student's t-test, Chi-square test, U-test by Mann and Whitney, Kruskal-Wallis test (H-test), Jonckheere-Terpstra test, and Wilcoxon test.

"Effective amount" includes an amount sufficient to ameliorate or prevent a symptom or sign of a medical condition. An effective amount also means an amount sufficient to allow or facilitate diagnosis. The effective amount for a subject may vary depending on the factors: such as the condition to be treated, the general health of the subject, the route and dose of administration, and the severity of side effects.

An effective amount can be the maximum dose or administration regimen that avoids significant side effects or toxic effects.

"Optional" or "optionally" means that the subsequently described event or circumstance may but need not occur, and that the description includes instances where the event or circumstance occurs and instances where it does not. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprising", "having", "including", and the like are to be understood in an inclusive sense, rather than an exclusive or exhaustive sense; that is to say, in the sense of "including but not limited to".

"Subject" or "patient" in the present disclosure means a mammal, particularly a primate, and especially a human.

**Example**

The present disclosure is further described below with reference to examples, but these examples are not intended to limit the scope of the present disclosure.

Experimental methods without specific conditions indicated in the examples or test examples of the present disclosure are generally conducted under conventional conditions, or under conditions recommended by the manufacturers of the starting materials or commercial products. See Sambrook et al., Molecular Cloning, A Laboratory Manual, Cold Spring Harbor Laboratory; Current Protocols in Molecular Biology, Ausubel et al., Greene Publishing Associates, Wiley Interscience, NY. Reagents for which the specific source is not specified are conventional, commercially available reagents.

**Example 1. Analysis of CTLA4 and TIGIT expression in tumour**

1. Single-cell sequencing analysis of CTLA4 and TIGIT co-expression in tumour patient samples

In this example, the inventors analysed the single-cell sequencing results from patient samples with various high-incidence cancers, including breast cancer (BRCA), colourectal cancer (CRC), and non-small cell lung cancer (NSCLC), in the TISCH database using bioinformatics. The data were sourced from public data sets included in the database, and were normalised using TPM (expression units were log (TPM/10+1)). The cell clustering was performed using the Louvain algorithm, and dimensionality reduction and visualisation analysis were performed using the UMAP algorithm. The inventors analysed the co-expression ratio and expression levels of TIGIT and CTLA4 on different intratumoural immune cells, as well as the difference in the co-expression ratio between intratumoural Tregs and peripheral Tregs.

The results are shown in FIG. 1A to FIG. 1D. The co-expression ratio of CTLA4 and TIGIT was high on intratumoural Tregs across various high-incidence cancers (FIG. 1A); the expression levels of CTLA4 and TIGIT were relatively comparable on intratumoural Tregs across various high-incidence cancers (FIB. 1B); among intratumoural immune cells, the co-expression ratio of CTLA4 and TIGIT was the highest on Tregs compared to that on other immune cells (FIG. 1C); and the co-expression ratio of CTLA4 and TIGIT on intratumoural Tregs was significantly higher than that on peripheral Tregs (FIG. 1D). These results from human tumour samples showed that CTLA4 and TIGIT had the characteristic of being selectively highly expressed and co-expressed on intratumoural Tregs.

2. Analysis of CTLA4 and TIGIT co-expression in CTLA4/TIGIT double-humanised mouse MC38 colon cancer model

Experimental method: CTLA4/TIGIT double-humanised mice (purchased from Biocytogen Pharmaceuticals (Beijing) Co., Ltd.) were acclimatised for 7 days. MC38 cells (purchased from Shunran (Shanghai) Biological Technology Co., Ltd.) were subcutaneously inoculated on the right side of the double-humanised mice at $5 \times 10^5$ cells/0.1 mL/mouse. Mouse body weight and tumour volume were measured twice weekly. Five mice with tumour sizes of 300-500 mm$^3$ were selected for euthanasia. Tumours were excised and photographed, and anticoagulated blood and tumours were collected for flow cytometry (flow cytometry panel: Live/dead, mCD45, mCD3, mCD4, mCD25, mFoxp3, hCTLA4, hTIGIT). The data were statistically analysed (Mean $\pm$ SEM), with $p < 0.05$ considered statistically significant.

The results are shown in FIG. 2. The expression level of TIGIT on Tregs in the tumour was significantly higher than that on Tregs in peripheral blood; the expression level of CTLA4 on Tregs in the tumour was significantly higher than that on Tregs in peripheral blood; and the co-expression ratio of CTLA4 and TIGIT on Tregs in the tumour was significantly higher than that on Tregs in peripheral blood. These results from the double-humanised mice also showed that CTLA4 and TIGIT were significantly highly expressed and co-expressed on Tregs in tumour tissue.

**Example 2. Design and preparation of anti-CTLA4/TIGIT antibody**

In this example, anti-CTLA4/TIGIT antibodies As-3 and S-15 were designed.

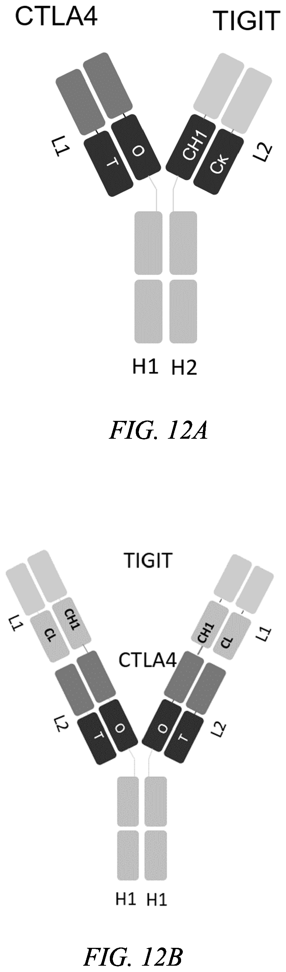

The As-3 molecule (as shown in FIG. 12A) consists of two heavy chains and two light chains. The CDRs used at the CTLA4 end are set forth in SEQ ID NOs: 1-6, and the heavy chain variable region (VH) and the light chain variable region (VL) are set forth in SEQ ID NOs: 13-14, respectively; and the CDRs used at the TIGIT end are set forth in SEQ ID NOs: 7-12, and the heavy chain variable region (VH) and the light chain variable region (VL) are set forth in SEQ ID NOs: 15-16, respectively.

To prevent or reduce heavy and light chain mispairing, the Obscurin-O and Titin-T, as set forth in SEQ ID NOs: 17-18, and the CH1 and Cκ, as set forth in SEQ ID NOs: 19-20, were used.

To prevent or reduce heavy chain mispairing, Fc1 and Fc2 containing KIH mutations were used, and DLE mutations were introduced to enhance ADCC, with the sequences of Fc1 and Fc2 as set forth in SEQ ID NOs: 25-26.

For As-3, the heavy chain sequences are set forth in SEQ ID NOs: 21 and 23, and the light chain sequences are set forth in SEQ ID NOs: 22 and 24.

The S-15 molecule (FIG. 12B) is a symmetric bispecific antibody that contains one heavy chain and two light chains. The CDRs used at the CTLA4 end are set forth in SEQ ID NOs: 27-32; and the CDRs used at the TIGIT end are set forth in SEQ ID NOs: 7-12. To prevent or reduce heavy and light chain mispairing, the Obscurin-O and Titin-T, as set forth in SEQ ID NOs: 17-18, and the CH1 and Cκ, as set forth in SEQ ID NOs: 19-20, were used. The heavy chain sequence of S-15 is set forth in SEQ ID NO: 33, the light chain sequence of TIGIT is set forth in SEQ ID NO: 34, and the light chain sequence of CTLA4 is set forth in SEQ ID NO: 35.

Table 2. CDRs of anti-CTLA4/TIGIT antibodies (Kabat numbering rule)

| Name | CDR sequence | | Sequence number |
|---|---|---|---|
| As-3 | HCDR1 | SYTMH | SEQ ID NO: 1 |
| | HCDR2 | FISYDGNNKYYADSVKG | SEQ ID NO: 2 |
| CTLA4 end | HCDR3 | TGWLGPFDY | SEQ ID NO: 3 |
| | LCDR1 | RASQSVGSSYLA | SEQ ID NO: 4 |
| | LCDR2 | GAFSRAT | SEQ ID NO: 5 |
| | LCDR3 | QQYGSSPWT | SEQ ID NO: 6 |
| As-3 TIGIT end | HCDR1 | NYWMH | SEQ ID NO: 7 |
| | HCDR2 | RIDPDSTGSKYNEKFKT | SEQ ID NO: 8 |
| | HCDR3 | EGAYGYYFDY | SEQ ID NO: 9 |
| | LCDR1 | RASENIYSYLA | SEQ ID NO: 10 |
| | LCDR2 | NARTLAE | SEQ ID NO: 11 |
| | LCDR3 | QYHSGSPLP | SEQ ID NO: 12 |
| S-15 CTLA4 end | HCDR1 | SYGMH | SEQ ID NO: 27 |
| | HCDR2 | VIWYDGSNKYADSVKG | SEQ ID NO: 28 |
| | HCDR3 | DPRGATLYYYYYGMDV | SEQ ID NO: 29 |
| | LCDR1 | RASQSINSYLD | SEQ ID NO: 30 |
| | LCDR2 | AASSLQS | SEQ ID NO: 31 |
| | LCDR3 | QQYYSTPFT | SEQ ID NO: 32 |
| S-15 TIGIT end | HCDR1 | NYWMH | SEQ ID NO: 7 |
| | HCDR2 | RIDPDSTGSKYNEKFKT | SEQ ID NO: 8 |
| | HCDR3 | EGAYGYYFDY | SEQ ID NO: 9 |
| | LCDR1 | RASENIYSYLA | SEQ ID NO: 10 |
| | LCDR2 | NARTLAE | SEQ ID NO: 11 |
| | LCDR3 | QYHSGSPLP | SEQ ID NO: 12 |

> As-3 VH1

QVQLVESGGGVVQPGRSLRLSCAASGFTFS<u>SYTMH</u>WVRQAPGKGLEWVT<u>FISYDGNNKYYADSVKG</u>RFTISRDNSKNTLYLQMNSLRAEDTAIYYCAR<u>TGWLGPFDY</u>WGQGTLVTVSS

(SEQ ID NO: 13)

> As-3 VL1

EIVLTQSPGTLSLSPGERATLSC<u>RASQSVGSSYLA</u>WYQQKPGQAPRLLIY<u>GAFSRAT</u>GIPDRFSGSGSGTDFTLTISRLEPEDFAVYYC<u>QQYGSSPWT</u>FGQGTKVEIK

(SEQ ID NO: 14)

> As-3 VH2

EVQLVQSGAEVKKPGASVKVSCKASGYTFT<u>NYWMH</u>WVRQAPGQGLEWMG<u>RIDPDSTGSKYNEKFKT</u>RVTMTRDTSTSTVYMELSSLRSEDTAVYYCAR<u>EGAYGYYFDY</u>WGQGTLVTVSS

(SEQ ID NO: 15)

> As-3 VL2

DIQMTQSPSSLSASVGDRVTITC<u>RASENIYSYLA</u>WYQQKPGKSPKLLIY<u>NARTLAEG</u>VPSRFSGSGSGTDFTLTISSLQPEDFATYYC<u>QYHSGSPLP</u>FGGGTKVEIK

(SEQ ID NO: 16)

>Obscurin-O

SGAPRFLTRPKASVVSVGKDATLSCQIVGNPFPQVSWEKDKQPVTAGVRFRLAQDGDLYRLKILDLQLSDSGQYVCRARNAHGEAFACLGLQVDAEA

(SEQ ID NO: 17)

>Titin-T

GIPPKIECLPIDISIDEGKVLTVASAFTGEPTPEVTWSTGGRKIHSQEQGRFHIENTDDSTTLTIKDVQKDGGLYTLTLRNEFGSDSATVNIHIRSI

(SEQ ID NO: 18)

> CH1

ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKS
C

(SEQ ID NO: 19)

> Cκ

RTVAAPSVFIFPPSDEQLKSGTASVVCLLNNFYPREAKVQWKVDNALQSG
NSQESVTEQDSKDSTYSLSSTLTLSKADYEKHKVYACEVTHQGLSSPVTKS
FNRGEC

(SEQ ID NO: 20)

> As-3 H1

QVQLVESGGGVVQPGRSLRLSCAASGFTFSSYTMHWVRQAPGKGLEWVT
FISYDGNNKYYADSVKGRFTISRDNSKNTLYLQMNSLRAEDTAIYYCART
GWLGPFDYWGQGTLVTVSSGGGGSSGAPRFLTRPKASVVSVGKDATLSC
QIVGNPFPQVSWEKDKQPVTAGVRFRLAQDGDLYRLKILDLQLSDSGQYV
CRARNAHGEAFACLGLQVDAE*DKTHTCPPCPAPELLGGPDVFLFPPKPK*

*DTLMISRTPEVTCVVVDVSHEDPEVKFNWYVDGVEVHNAKTKPREEQYNSTYR*
*VVSVLTVLHQDWLNGKEYKCKVSNKALPLPEEKTISKAKGQPREPQVYTLPPCR*
*EEMTKNQVSLWCLVKGFYPSDIAVEWESNGQPENNYKTTPPVLDSDGSFFLYS*
*KLTVDKSRWQQGNVFSCSVMHEALHNHYTQKSLSLSPGK*

(SEQ ID NO: 21)

> As-3 L1

EIVLTQSPGTLSLSPGERATLSCRASQSVGSSYLAWYQQKPGQAPRLLIYG
AFSRATGIPDRFSGSGSGTDFTLTISRLEPEDFAVYYCQQYGSSPWTFGQGT
KVEIKGGGGSGIPPKIECLPIDISIDEGKVLTVASAFTGEPTPEVTWSTGGRKI
HSQEQGRFHIENTDDSTTLTIKDVQKDGGLYTLTLRNEFGSDSATVNIHIR
SI

(SEQ ID NO: 22)

> As-3 H2

EVQLVQSGAEVKKPGASVKVSCKASGYTFTNYWMHWVRQAPGQGLEW
MGRIDPDSTGSKYNEKFKTRVTMTRDTSTSTVYMELSSLRSEDTAVYYCA
REGAYGYYFDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQ
TYICNVNHKPSNTKVDKKVEPKS*DKTHTCPPCPAPELLGGPDVFLFPPKP*
*KDTLMISRTPEVTCVVVDVSHEDPEVKFNWYVDGVEVHNAKTKPREEQYNSTY*
*RVVSVLTVLHQDWLNGKEYKCKVSNKALPLPEEKTISKAKGQPREPQVCTLPPS*
*REEMTKNQVSLSCAVKGFYPSDIAVEWESNGQPENNYKTTPPVLDSDGSFFLVS*
*KLTVDKSRWQQGNVFSCSVMHEALHNHYTQKSLSLSPGK*

(SEQ ID NO: 23)

> As-3 L2

DIQMTQSPSSLSASVGDRVTITCRASENIYSYLAWYQQKPGKSPKLLIYNA
RTLAEGVPSRFSGSGSGTDFTLTISSLQPEDFATYYCQYHSGSPLPFGGGTK
VEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPV
TKSFNRGEC

(SEQ ID NO: 24)

> IgG1 Fc1 (S354C, T366W, S239D/A330L/I332E)

DKTHTCPPCPAPELLGGPDVFLFPPKPKDTLMISRTPEVTCVVVDVSHEDPE
VKFNWYVDGVEVHNAKTKPREEQYNSTYRVVSVLTVLHQDWLNGKEYK

CKVSNKALPLPEEKTISKAKGQPREPQVYTLPPCREEMTKNQVSLWCLVK
GFYPSDIAVEWESNGQPENNYKTTPPVLDSDGSFFLYSKLTVDKSRWQQG
NVFSCSVMHEALHNHYTQKSLSLSPGK      (SEQ ID NO: 25)

> IgG1 Fc2 (Y349C, T366S, L368A, Y407V, S239D/A330L/I332E)

DKTHTCPPCPAPELLGGPDVFLFPPKPKDTLMISRTPEVTCVVVDVSHEDPE
VKFNWYVDGVEVHNAKTKPREEQYNSTYRVVSVLTVLHQDWLNGKEYK
CKVSNKALPLPEEKTISKAKGQPREPQVCTLPPSREEMTKNQVSLSCAVKG
FYPSDIAVEWESNGQPENNYKTTPPVLDSDGSFFLVSKLTVDKSRWQQGN
VFSCSVMHEALHNHYTQKSLSLSPGK      (SEQ ID NO: 26)

> S-15 H1 ( S239D/A330L/I332E )

EVQLVQSGAEVKKPGASVKVSCKASGYTFTNYWMHWVRQAPGQGLEW
MGRIDPDSTGSKYNEKFKTRVTMTRDTSTSTVYMELSSLRSEDTAVYYCA
REGAYGYYFDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQ
TYICNVNHKPSNTKVDKKVEPKSCDGGGGSGGGGSGGGGSQVQLVESGG
GVVQPGRSLRLSCAASGFTFSSYGMHWVRQAPGKGLEWVAVIWYDGSNK
YYADSVKGRFTISRDNSKNTLYLQMNSLRAEDTAVYYCARDPRGATLYY
YYYGMDVWGQGTTVTVSSGGGGSSGAPRFLTRPKASVVSVGKDATLSCQ
IVGNPFPQVSWEKDKQPVTAGVRFRLAQDGDLYRLKILDLQLSDSGQYVC
RARNAHGEAFACLGLQVDAE*DKTHTCPPCPAPELLGGPDVFLFPPKPKDT*
*LMISRTPEVTCVVVDVSHEDPEVKFNWYVDGVEVHNAKTKPREEQYNSTYRVV*
*SVLTVLHQDWLNGKEYKCKVSNKALPLPEEKTISKAKGQPREPQVYTLPPSREE*
*MTKNQVSLTCLVKGFYPSDIAVEWESNGQPENNYKTTPPVLDSDGSFFLYSKLT*
*VDKSRWQQGNVFSCSVMHEALHNHYTQKSLSLSPGK*
(SEQ ID NO: 33)

> S-15 L1

DIQMTQSPSSLSASVGDRVTITCRASENIYSYLAWYQQKPGKSPKLLIYNA
RTLAEGVPSRFSGSGSGTDFTLTISSLQPEDFATYYCQYHSGSPLPFGGGTK
VEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPV
TKSFNRGEC
(SEQ ID NO: 34)

> S-15 L2

DIQMTQSPSSLSASVGDRVTITCRASQSINSYLDWYQQKPGKAPKLLIYAA
SSLQSGVPSRFSGSGSGTDFTLTISSLQPEDFATYYCQQYYSTPFTFGPGTKV
EIKGGGGSGIPPKIECLPIDISIDEGKVLTVASAFTGEPTPEVTWSTGGRKIHS
QEQGRFHIENTDDSTTLTIKDVQKDGGLYTLTLRNEFGSDSATVNIHIRSI
(SEQ ID NO: 35)

In the above sequences, CDRs are underlined with straight lines, linkers are underlined with wavy lines, and Fc regions are presented in italics.

The polynucleotide sequences encoding the antibodies were cloned into a pTT5-containing vector and then transfected into CHOK1 cells. Expression and purification were performed using conventional methods, and the antibodies of interest, As-3 and S-15, were obtained after detection.

The sequences of the control antibodies used in the present disclosure are as follows:

> Anti-CTLA4 antibody Ipi heavy chain

QVQLVESGGGVVQPGRSLRLSCAASGFTFS<u>SYTMH</u>WVRQAPGKGLEWVT<u>FISYDGNNKYYADSVK</u>GRFTISRDNSKNTLYLQMNSLRAEDTAIYYCAR<u>TGWLGPFDY</u>WGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

(SEQ ID NO: 36)

> Anti-CTLA4 antibody Ipi light chain

EIVLTQSPGTLSLSPGERATLSC<u>RASQSVGSSYLA</u>WYQQKPGQAPRLLIY<u>GAFSRAT</u>GIPDRFSGSGSGTDFTLTISRLEPEDFAVYYC<u>QQYGSSPWT</u>FGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

(SEQ ID NO: 37)

> Anti-TIGIT antibody S2 heavy chain

EVQLVQSGAEVKKPGASVKVSCKASGYTFT<u>NYWMH</u>WVRQAPGQGLEWMG<u>RIDPDSTGSKYNEKFKT</u>RVTMTRDTSTSTVYMELSSLRSEDTAVYYCAR<u>EGAYGYYFDY</u>WGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

(SEQ ID NO: 38)

> Anti-TIGIT antibody S2 light chain

DIQMTQSPSSLSASVGDRVTITCRASENIYSYLAWYQQKPGKSPKLLIYNARTLAEGVPSRFSGSGSGTDFTLTISSLQPEDFATYYCQYHSGSPLPFGGGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

(SEQ ID NO: 39)

> Anti-CTLA4 antibody Tre heavy chain

QVQLVESGGGVVQPGRSLRLSCAASGFTFSSYGMHWVRQAPGKGLEWVAVIWYDGSNKYYADSVKGRFTISRDNSKNTLYLQMNSLRAEDTAVYYCARDPRGATLYYYYGMDVWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

(SEQ ID NO: 40)

Anti-CTLA4 antibody Tre light chain

DIQMTQSPSSLSASVGDRVTITCRASQSINSYLDWYQQKPGKAPKLLIYAASSLQSGVPSRFSGSGSGTDFTLTISSLQPEDFATYYCQQYYSTPFTFGPGTKV

EIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

(SEQ ID NO: 41)

> Anti-TIGIT antibody RG6058 heavy chain

EVQLQQSGPGLVKPSQTLSLTCAISGDSVSSNSAAWNWIRQSPSRGLEWLG KTYYRFKWYSDYAVSVKGRITINPDTSKNQFSLQLNSVTPEDTAVFYCTRE STTYDLLAGPFDYWGQGTLVTVSSASTKGPSVFPLAPSSKSTSGGTAALGC LVKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPPK PKDTLMISRTPEVTCVVVDVSHEDPEVKFNWYVDGVEVHNAKTKPREEQ YNSTYRVVSVLTVLHQDWLNGKEYKCKVSNKALPAPIEKTISKAKGQPRE PQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPG K

(SEQ ID NO: 42)

> Anti-TIGIT antibody RG6058 light chain

DIVMTQSPDSLAVSLGERATINCKSSQTVLYSSNNKKYLAWYQQKPGQPP NLLIYWASTRESGVPDRFSGSGSGTDFTLTISSLQAEDVAVYYCQQYYSTP FTFGPGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVT HQGLSSPVTKSFNRGEC                    (SEQ ID NO:43).

**Example 3. Detection of antigen-binding affinity of anti-CTLA4/TIGIT antibody**

1. Biacore detection

The affinity kinetics of the interaction between anti-CTLA4/TIGIT antibody and human TIGIT and human CTLA4 antigens were determined by surface plasmon resonance (SPR) technology using a Biacore biomacromolecular interaction instrument.

Experimental method:

The affinity kinetic analysis of the interaction between anti-CTLA4/TIGIT antibody and human CTLA4 (Acro Biosystems, Cat#CT4-H5229) and human TIGIT (Acro Biosystems Cat#TIT-H52H5) antigens was performed using Protein A biosensor chips (GE Healthcare, Cat#29127556). The test antibody at a certain concentration, prepared using HBS-EP+buffer solution, was used as the ligand, and captured on the Fc2 (Flow cell 2, Fc2) channel of the chip at a flow rate of 10 μL/min for a capture time of 30 s. CTLA4 and TIGIT at a certain concentration, prepared using HBS-EP+buffer solution, were used as analytes, and injected into the reference channel Fc2 (Flow cell 1, Fc1) and the detection channel Fc2 of the chip. The analytes were prepared using HBS-EP+buffer solution, and subjected to 2-fold gradient dilution across 7 concentration points. The flow rate was 30 μL/min, the association time was 120 seconds, and the dissociation time was 1200 seconds. Finally, regeneration was performed using 10 mM Glycine pH 1.5 (GE Healthcare, Cat#BR-1003-54). The regeneration conditions were as follows: regeneration time: 30 s, flow rate: 30 μL/min.

The affinity kinetic analysis of the interaction between human CTLA4 and human TIGIT antigens and anti-CTLA4/TIGIT antibody was performed using a CM5 chip. According to the instructions of the His capture kit (Cytiva, Cat#29234602), an anti-histidine antibody was covalently coupled onto the Fc1 and Fc2 channels of a CM5 biosensor chip (GE Healthcare, Cat#29149603), and human CTLA4 or human TIGIT antigen was used as the ligand and captured on the Fc2 (Flow cell 2, Fc2) channel of the chip at a flow rate of 10 μL/min for a capture time of 30 s. Monovalent anti-CTLA4/TIGIT antibody, prepared using HBS-EP+buffer solution, was used as the analyte. The analyte was subjected to 2-fold gradient dilution across 7 concentration points. The flow rate was 30 µL/min, the association time was 100 seconds, and the dissociation time was 1800 seconds. Finally, regeneration was performed using 10 mM Glycine pH 1.5 (GE Healthcare, BR-1003-54). The regeneration conditions were as follows: regeneration time: 30 s, flow rate: 30 µL/min.

Data statistics and analysis: SPR signals were acquired and saved using Biacore 8K Control Software, and subsequent data processing was performed using Biacore 8K Evaluation analysis software.

Experimental results: The association and dissociation constants of AS-3 with human CTLA4 or TIGIT antigen are shown in Table 3-1 (1 : 1 binding). The results showed that the single-arm affinity of AS-3 was relatively consistent with that of the anti-CTLA4 antibody Ipi and the anti-TIGIT antibody S2.

The association and dissociation constants for the interaction between human CTLA4 or human TIGIT antigen and anti-CTLA4/TIGIT antibody are shown in Table 3-2 (1 : 1 binding). The results showed that due to the single-arm design, AS-3 exhibited reduced binding capacity to CTLA4 or TIGIT antigen compared to the dual-arm anti-CTLA4 antibody Ipi and the dual-arm anti-TIGIT antibody S2.

Table 3-1. Binding affinity of anti-CTLA4/TIGIT antibody to antigens CTLA4 and TIGIT

| Ligand | Analyte | ka (1/Ms) | kd (1/s) | $K_D$ (M) |
|--------|---------|-----------|----------|-----------|
| As-3 | Human CTLA4 | 1.53E+05 | 1.66E-03 | 1.08E-08 |
| Ipi | | 1.45E+05 | 1.31E-03 | 9.01E-09 |
| As-3 | Human TIGIT | 1.73E+06 | 7.13E-05 | 4.11E-11 |
| S2 | | 1.05E+06 | 1.09E-04 | 1.04E-10 |

Table 3-2. Binding affinity of antigens CTLA4 and TIGIT to anti-CTLA4/TIGIT antibody

| Ligand | Analyte | ka (1/Ms) | kd (1/s) | $K_D$ (M) |
|--------|---------|-----------|----------|-----------|
| Human CTLA4 | As-3 | 4.57E+05 | 3.20E-03 | 7.00E-09 |
| | Ipi | 9.72E+05 | <1E-05 | <1E-11 |
| Human TIGIT | As-3 | 9.72E+05 | 5.20E-04 | 5.35E-10 |
| | S2 | 1.61E+06 | <1E-05 | <6.2E-12 |

2. FACS detection

The binding activity of anti-CTLA4/TIGIT antibody to human CTLA4 and human TIGIT proteins expressed on the cell surface was detected using FACS experiments.

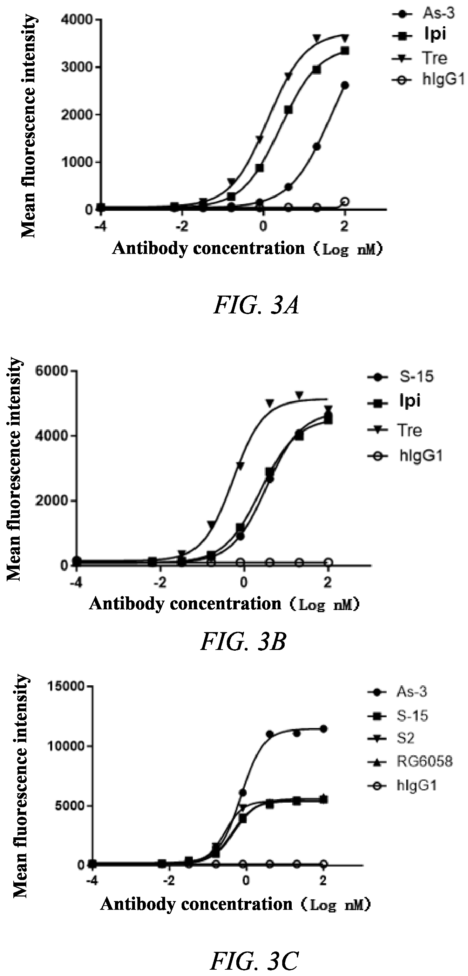

Experimental method: CHOK1-CTLA4 cells were obtained by stable transfection of CHOK1 cells to express human CTLA4 protein, and CHOK1-TIGIT cells were obtained by stable transfection of CHOK1 cells to express human TIGIT protein. Cell culture media for both cells were F12K (Gibco, Cat#21127-022) containing 10% inactivated foetal bovine serum (Gibco, Cat#10091-148). The buffer was sterile PBS (containing 2% foetal bovine serum). The CHOK1-CTLA4 or CHOK1-TIGIT cells were washed twice with the buffer, and then the cells were seeded into 96-well U-bottom plates at $1 \times 10^5$ cells/well. Gradient-diluted test samples were added. The cells were incubated at 4°C for 1 hour, and then washed twice with the test buffer. Subsequently, an Alexa Fluor 647-labelled goat antihuman (IgG, Fcγ fragment specific) antibody (Jackson ImmunoResearch, Cat#109-605-098) was added. The cells were incubated at 4°C for 30 minutes, and washed twice with the test buffer, and then fluorescence signals were read using a flow cytometer. The results are shown in FIG. 3A to FIG. 3C, Table 3-3, Table 3-4, and Table 3-5.

Experimental results: As-3 exhibited binding capacity to the CTLA4 protein on the surface of CHOK1-CTLA4 cells, and due to the single-arm design, its affinity for CTLA4 was weaker compared to that of Ipi and Tre; As-3 exhibited good binding capacity to the TIGIT protein on the surface of CHOK1-TIGIT cells, which was comparable to that of the anti-TIGIT antibodies S2 and RG6058; and the binding capacity of S-15 to the CTLA4 on the surface of CHOK1-CTLA4 cells was comparable to that of Tre, and the binding capacity of S-15 to the TIGIT protein on the surface of CHOK1-TIGIT cells was comparable to that of the anti-TIGIT antibody S2. Among these, hIgG1 was the isotype control antibody.

Table 3-3. Results of As-3 binding to CHOK1-CTLA4 cells

| Designation of antibody | $EC_{50}$ (nM) | Maximum fluorescence value |
|-------------------------|----------------|----------------------------|
| As-3 | 47.12 | 3986 |

(continued)

| Designation of antibody | EC$_{50}$ (nM) | Maximum fluorescence value |
|---|---|---|
| Ipi | 2.58 | 3433 |
| Tre | 1.27 | 3736 |
| hIgG1 | N.A. | N.A. |

Table 3-4. Results of S-15 binding to CHOK1-CTLA4 cells

| Designation of antibody | EC$_{50}$ (nM) | Maximum fluorescence value |
|---|---|---|
| S-15 | 3.35 | 4747 |
| Ipi | 2.43 | 4553 |
| Tre | 0.53 | 5142 |
| hIgG1 | N.A. | N.A. |

Table 3-5. Results of anti-CTLA4/TIGIT antibody binding to CHOK1-TIGIT cells

| Designation of antibody | EC$_{50}$ (nM) | Maximum fluorescence value |
|---|---|---|
| As-3 | 0.72 | 11465 |
| S-15 | 0.43 | 5425 |
| S2 | 0.27 | 5431 |
| RG6058 | 0.48 | 5598 |
| hIgG1 | N.A. | N.A. |
| N.A. indicates that EC$_{50}$ or IC$_{50}$ could not be obtained by fitting. | | |

**Example 4. Detection of activity of anti-CTLA4/TIGIT antibody in blocking ligand binding to antigens on cells**

In this example, FACS experiments were used to detect whether the anti-CTLA4/TIGIT antibody blocked the binding of CTLA4 to CD80 expressed on the cell surface, and whether it blocked the binding of TIGIT to CD155 expressed on the cell surface.

Experimental method: CHOK1-CTLA4 cells were obtained by stable transfection of CHOK1 cells to express human CTLA4 protein, CHOK1-TIGIT cells were obtained by stable transfection of CHOK1 cells to express human TIGIT protein, CHOK1-CD80 cells were obtained by stable transfection of CHOK1 cells to express human CD80 protein, and CHOK1-CD155 cells were obtained by stable transfection of CHOK1 cells to express human CD155 protein. Cell culture media for all cells were F12K (Gibco, Cat#21127-022) containing 10% inactivated foetal bovine serum (Gibco, Cat#10091-148). The buffer was sterile PBS (containing 2% foetal bovine serum). The CHOK1-CTLA4, CHOK1-TIGIT, CHOK1-CD80, and CHOK1-CD155 cells were washed twice with the buffer, respectively. Antigen-expressing cells and ligand-expressing cells were labelled with CFSE (Invitrogen, Cat#C34554) and Far Red (Invitrogen, Cat#C34564), respectively. After labelling, excess fluorescent dyes were washed away with the test buffer. $1 \times 10^5$ antigen-expressing cells and $1 \times 10^5$ corresponding ligand-expressing cells were seeded into 96-well U-bottom plates. Gradient-diluted test samples were added. The cells were incubated at 37°C for 2 hours, and then fluorescence signal values were read using a flow cytometer.

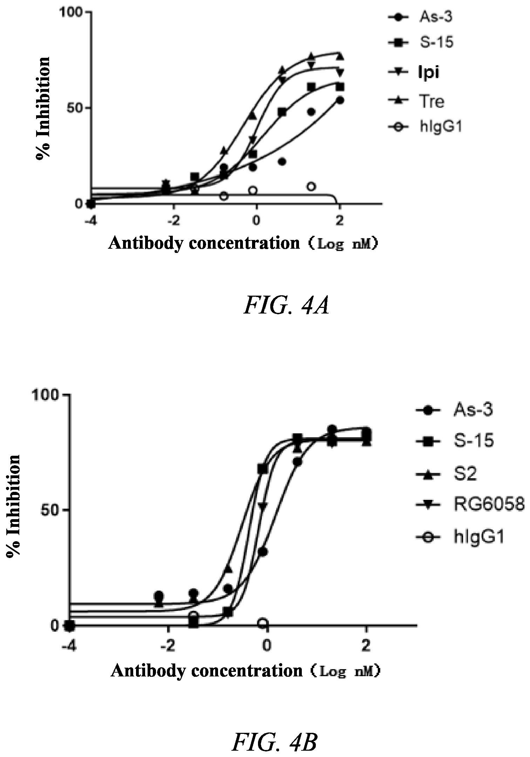

Experimental results: see FIG. 4A and FIG. 4B, and Table 4-1, and Table 4-2. The FACS detection results showed: As-3 exhibited a blocking effect against the binding of the CHOK1-CTLA4 cells to the CHOK1-CD80 cells, and due to the single-arm design, the blocking effect was weaker than that of Ipi and Tre; As-3 exhibited a good blocking effect against the binding of the CHOK1-TIGIT cells to the CHOK1-CD155 cells, which was comparable to that of anti-TIGIT antibodies S2 and RG6058; the blocking effect of S-15 against the binding of the CHOK1-CTLA4 cells to the CHOK1-CD80 cells was comparable to that of Tre; and the blocking effect of S-15 against the binding of the CHOK1-TIGIT cells to the CHOK1-CD155 cells was also comparable to that of the anti-TIGIT antibody S2. Since Ipilimumab and Tremelimumab might induce CTLA4-characteristic toxicities clinically, the inventors selected As-3, which has weaker blocking activity, for further validation.

Table 4-1. Results of anti-CTLA4/TIGIT antibody blocking CTLA4-CD80 binding

| Designation of antibody | IC$_{50}$ (nM) | Maximum inhibition rate |
|---|---|---|
| As-3 | N.A. | N.A. |
| S-15 | 1.42 | 66.01% |
| Ipi | 1.01 | 71.08% |
| Tre | 0.49 | 79.81% |
| hIgG1 | N.A. | N.A. |

Table 4-2. Results of anti-CTLA4/TIGIT antibody blocking TIGIT-CD155 binding

| Designation of antibody | IC$_{50}$ (nM) | Maximum inhibition rate |
|---|---|---|
| As-3 | 1.44 | 85.94% |
| S-15 | 0.42 | 81.08% |
| S2 | 0.31 | 80.84% |
| RG6058 | 0.67 | 80.26% |
| hIgG1 | N.A. | N.A. |
| N.A. indicates that EC$_{50}$ or IC$_{50}$ could not be obtained by fitting. | | |

**Example 5. Detection of binding capacity and avidity effect of anti-CTLA4/TIGIT antibody to double-positive cells co-expressing CTLA4 and TIGIT**

1. The binding activity of anti-CTLA4/TIGIT antibody to single-positive cells expressing human CTLA4 and to double-positive cells co-expressing CTLA4 and TIGIT was detected in FACS experiments.

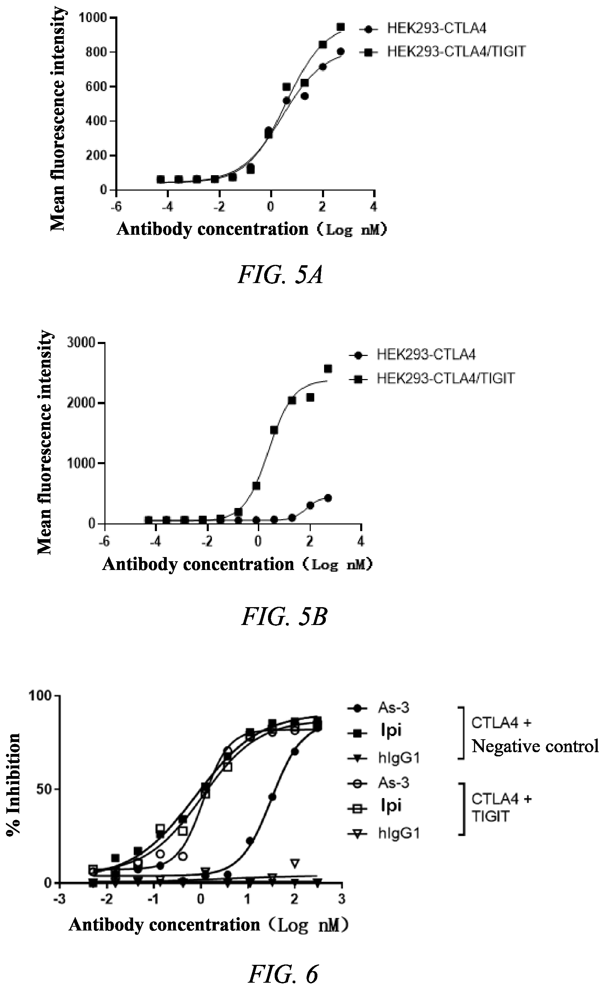

Experimental method: HEK293-CTLA4 cells were obtained by stable transfection of HEK293 cells to express human CTLA4 protein, and HEK293-CTLA4/TIGIT cells were obtained by stable transfection of HEK293-CTLA4 cells to express human TIGIT protein. Cell culture media for both cells were DMEM (Gibco, Cat#11995-065) containing 10% inactivated foetal bovine serum (Gibco, Cat#10091-148). The HEK293-CTLA4 or HEK293-CTLA4/TIGIT cells were washed twice with a buffer (PBS containing 2% foetal bovine serum). The cells were seeded into 96-well U-bottom plates at $1 \times 10^5$ cells/well. Gradient-diluted test samples were added. The cells were incubated at 4°C for 1 hour, and then washed twice with the test buffer. Subsequently, an Alexa Fluor 647-labelled goat antihuman (IgG, Fc$\gamma$ fragment specific) antibody (Jackson ImmunoResearch, Cat#109-605-098) was added. The cells were incubated at 4°C for 30 minutes, and washed twice with the test buffer, and then fluorescence signals were read using a flow cytometer.
Experimental results: see FIG. 5A and FIG. 5B, and Table 5. The binding activity of As-3 to double-positive cells was better than that to HEK293-CTLA4 single-positive cells, whereas the binding activity of Ipi to double-positive cells was comparable to that to single-positive cells, indicating that As-3 enhances its binding capacity to cells co-expressing CTLA4 and TIGIT via an avidity effect.

Table 5. Results of binding activity of anti-CTLA4/TIGIT antibody to HEK293-CTLA4 single-positive cells and to HEK293-CTLA4/TIGIT double-positive cells

| Designation of antibody | HEK293-CTLA4 | | HEK293-CTLA4/TIGIT | |
|---|---|---|---|---|
| | EC$_{50}$ (nM) | Maximum fluorescence value | EC$_{50}$ (nM) | Maximum fluorescence value |
| As-3 | 70.44 | 447 | 2.45 | 2394 |
| Ipi | 2.82 | 822 | 3.88 | 974 |

2. The blocking capacity of the anti-CTLA4/TIGIT antibody against CTLA4-CD80 in the presence of TIGIT protein was detected using an ELISA assay.
Experimental method: Two groups were set up for comparison. In the first group, a 96-well plate (Costar, Cat# 3590) was simultaneously coated with CTLA4 protein (Acro Biosystems, Cat# CT4-H5229) and an irrelevant protein. In the second group, a 96-well plate was simultaneously coated with CTLA4 protein and TIGIT protein (Acro Biosystems, Cat# TIT-H52H5), maintaining the same concentration as in the first group. The plates in both groups were incubated at 4°C overnight, and then washed three times. 3% BSA was then added, and the plates were incubated at room temperature for 1 hour, and then washed three times. A constant concentration of biotin-labelled CD80 protein (AMSBIO, Cat# 71114-1) and gradient-diluted test antibodies were added. The plates were incubated at room temperature for 2 hours, and then washed three times. SA-HRP (Jackson ImmunoResearch, Cat# 016-030-084) was added, and the plates were incubated at room temperature for 1 hour, and then washed six times. A chromogenic substrate was added, and the OD450 values were read using EnVision.

Experimental results: see FIG. 6 and Table 6. ELISA assay results showed: under the condition of simultaneous coating with CTLA4 and an irrelevant protein, the blocking effect of As-3 against CTLA4-CD80 was weaker than that of Ipi, and under the condition of simultaneous coating with CTLA4 and TIGIT proteins, the blocking activity of As-3 was comparable to that of Ipi, indicating that in the simultaneous presence of CTLA4 and TIGIT, As-3 enhances its blocking activity against CTLA4-CD80 via an avidity effect, thereby attenuating the immunosuppressive effect of CTLA4.

Table 6. Results of blocking activity of anti-CTLA4/TIGIT antibody against CTLA4-CD80

| Designation of antibody | CTLA4+negative control | | CTLA4+TIGIT | |
|---|---|---|---|---|
| | $IC_{50}$ (nM) | Maximum inhibition rate | $IC_{50}$ (nM) | Maximum inhibition rate |
| As-3 | 31.90 | 86.08% | 1.14 | 81.94% |
| Ipi | 0.77 | 90.85% | 1.06 | 87.27% |

**Example 6. Detection of anti-CTLA4/TIGIT antibody-mediated ADCC killing effect and the antibody's ability to deplete Tregs via ADCC**

1. The anti-CTLA4/TIGIT antibody-mediated ADCC killing activity of NK cells against single-positive cells expressing CTLA4 and double-positive cells co-expressing CTLA4 and TIGIT was evaluated using a lactate dehydrogenase (LDH) assay.

Experimental method: Cryopreserved PBMCs were purchased from Shanghai Saily Biotechnology Co., Ltd. (the same below). After thawing, the cells were cultured in medium RPMI1640 (Gibco, Cat#10491A-01) containing 10% inactivated foetal bovine serum (Gibco, Cat#10091-148), and incubated at 37°C overnight. On the next day, the single-positive and double-positive cells were collected and resuspended in phenol red-free RPMI1640 (Gibco, Cat#11835-030) containing 2% foetal bovine serum, and the cell density was adjusted to $2 \times 10^5$ cells/mL. Subsequently, the cells were seeded into 96-well plates at 50 $\mu$L/well, followed by the addition of 50 $\mu$L of gradient-diluted test antibodies. The PBMCs were collected and resuspended in phenol red-free RPMI1640 (Gibco, Cat# 11835-030) containing 2% foetal bovine serum, and the cell density was adjusted to $2 \times 10^6$ cells/mL. Subsequently, the cells were seeded into the aforementioned test plates at 100 $\mu$L/well, and the cells were incubated in an incubator at 37°C and 5% CO2 for 4 hours. The cell culture plates were removed and centrifuged (400 g, 5 minutes) to collect the cell culture supernatant. The level of LDH was detected using a CytoTox 96® Non-Radioactive Cytotoxicity Assay kit.

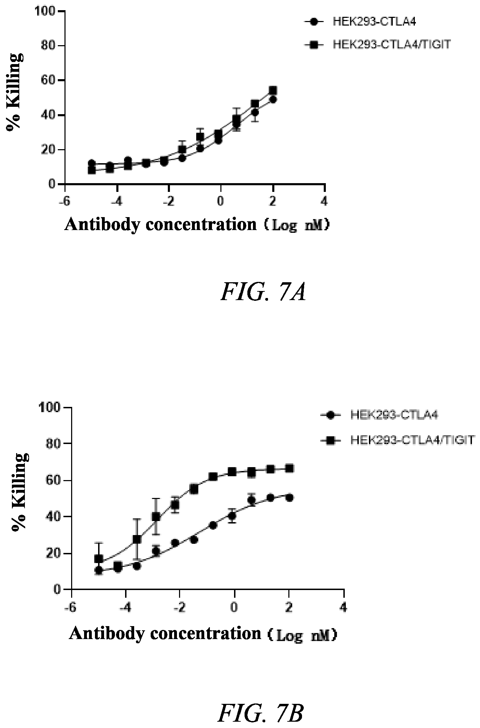

Experimental results: see FIG. 7A and FIG. 7B, and Table 7. The As-3-mediated killing activity against HEK293-CTLA4/TIGIT double-positive cells was superior to that against HEK293-CTLA4 single-positive cells, whereas the Ipi-mediated killing activity against double-positive cells was comparable to that against single-positive cells, indicating that As-3 enhances its selective killing capacity against cells co-expressing CTLA4 and TIGIT.

Table 7. Results of anti-CTLA4/TIGIT antibody-mediated ADCC killing activity of NK against HEK293-CTLA4 single-positive cells and HEK293-CTLA4/TIGIT double-positive cells

| Designation of antibody | HEK293-CTLA4 | | HEK293-CTLA4/TIGIT | |
|---|---|---|---|---|
| | $EC_{50}$ (nM) | Maximum killing rate | $EC_{50}$ (nM) | Maximum killing rate |
| As-3 | 0.054 | 56.56% | 0.0015 | 66.71% |
| Ipi | N.A. | N.A. | N.A. | N.A. |
| N.A. indicates that $EC_{50}$ or $IC_{50}$ could not be obtained by fitting. | | | | |

2. The anti-CTLA4/TIGIT antibody-mediated ADCC killing activity of NK cells against Treg cells was evaluated using a lactate dehydrogenase (LDH) assay.

Experimental method: Treg cells were first induced. CD4+ T cells were isolated from fresh PBMCs using an EasySep™ Human CD4+ T Cell Enrichment Kit (STEMCELL, CAT#19052). The CD4+ T cells were adjusted to a density of $2.5 \times 10^6$ cells/mL using RPMI 1640 (Gibco, Cat#10491A-01) containing 10% inactivated foetal bovine serum (Gibco, Cat#10091-148), with a total volume of 20 mL. The following components were added: loaded Anti-Biotin MACSiBead Particles (Miltenyi Biotec, Cat#130-091-441) (Bead : cell=1 : 1), human IL-2 (PEPROTECH, Cat#200-02-50UG) (400 U/mL), rapamycin (SELLECK, Cat#S1039) (100 ng/mL), TGFβ (PEPROTECH, Cat#100-21) (20 ng/mL), and atRA (Sigma, Cat#R2625-100MG) (100 nM). The cells were incubated at 37°C and 5% CO2 for 3 days. The cell density was adjusted to $1.2\text{-}2.5 \times 10^6$ cells/mL, 20 U/mL human IL-2 was supplemented, and the cells were incubated at 37°C and 5% $CO_2$ for 3 days. According to the T Cell Activation/Expansion Kit (Miltenyi Biotec, Cat#130-091-441), the Anti-Biotin MACSiBead Particles were removed. Subsequently, CD4+CD25+ T cells were isolated and purified, and beads were removed using a Dynabeads® Regulatory CD4+CD25+ T Cell Kit (life, Cat#11363D), finally obtaining bead-free induced Treg (referred to as iTreg) cells. The antibody-mediated ADCC killing activity of NK cells against iTreg cells was evaluated using a lactate dehydrogenase (LDH) assay. Cryopreserved PBMCs were thawed and then cultured in medium RPMI1640 (Gibco, Cat#10491A-01) containing 10% inactivated foetal bovine serum (Gibco, Cat#10091-148), and incubated at 37°C overnight. On the next day, the iTreg cells were collected and resuspended in phenol red-free RPMI1640 (Gibco, Cat#11835-030) containing 2% foetal bovine serum, and the cell density was adjusted to $1 \times 10^5$ cells/mL. Subsequently, the cells were seeded into 96-well plates at 50 μL/well, followed by the addition of 50 μL of gradient-diluted test antibodies. The PBMCs were collected and resuspended in phenol red-free RPMI1640 (Gibco, Cat#11835-030) containing 2% foetal bovine serum, and the cell density was adjusted to $5 \times 10^6$ cells/mL. Subsequently, the cells were seeded into the aforementioned test plates at 100 μL/well, and the cells were incubated in an incubator at 37°C and 5% CO2 for 4 hours. The cell culture plates were removed and centrifuged (400 g, 5 minutes) to collect the cell culture supernatant. The level of LDH was detected using a CytoTox 96® Non-Radioactive Cytotoxicity Assay kit.

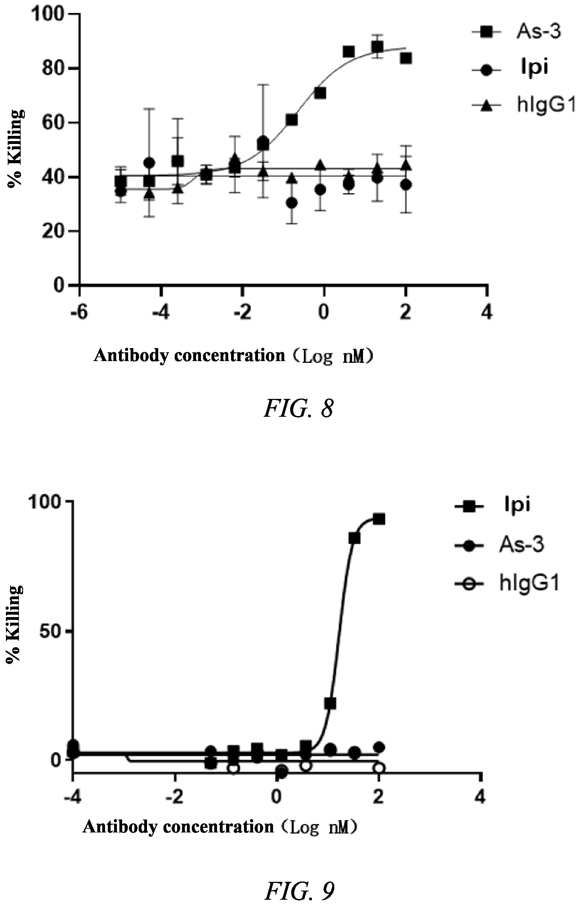

Experimental results: see FIG. 8, and Table 8. The ADCC detection results showed that the As-3-mediated ADCC killing activity against iTreg was stronger than that mediated by Ipi.

Table 8. Results of anti-CTLA4/TIGIT antibody-mediated ADCC killing activity of NK against iTreg

| Designation of antibody | iTreg | |
|---|---|---|
| | $EC_{50}$ (nM) | Maximum killing rate |
| As-3 | 0.23 | 88.01 % |
| Ipi | N.A. | N.A. |
| N.A. indicates that $EC_{50}$ or $IC_{50}$ could not be obtained by fitting. | | |

### Example 7. Detection of anti-CTLA4/TIGIT antibody-mediated CDC effect

The anti-CTLA4/TIGIT antibody-mediated CDC effect activity was evaluated using CTG detection.

Experimental method: CHOK1-CTLA4 cells were obtained by stable transfection of CHOK1 cells to express human CTLA4 protein. A cell culture medium was F12K (Gibco, Cat#21127-022) containing 10% inactivated foetal bovine serum (Gibco, Cat#10091-148). The CHOK1-CTLA4 cells were collected and resuspended in phenol red-free RPMI1640 (Gibco, Cat#11835-030) containing 2% foetal bovine serum, and the cell density was adjusted to $2 \times 10^5$ cells/mL. Subsequently, the cells were seeded into 96-well plates at 50 μL/well, followed by the addition of 40 μL of gradient-diluted test antibodies and 10 μL of complement (QUIDEL, Cat#A1 13). The resulting mixture was incubated in an incubator at 37°C and 5% CO2 overnight. The cell culture plates were removed. Triton was added to the positive control wells to lyse the cells. Subsequently, 50 μL of CTG (Promega, Cat#PR-G7573) was added to each well. The cells were incubated at a room temperature for 10 minutes, and the fluorescence values were read using Envision. The results are shown in FIG. 9 and Table 9.

The CDC detection results showed that the CDC effect of the CTLA4/TIGIT antibody was significantly weaker than that of Ipi, indicating that the anti-CTLA4/TIGIT antibody, through DLE mutations, reduces the CDC effect, which may mitigate the toxic side effects (such as hypophysitis) of CTLA4 antibodies in the clinic and improve safety.

Table 9. Results of anti-CTLA4/TIGIT antibody-mediated CDC effect

| Designation of antibody | iTreg | |
|---|---|---|
| | $EC_{50}$ (nM) | Maximum killing rate |
| As-3 | N.A. | N.A. |
| Ipi | 1.22 | 93.87% |
| N.A. indicates that $EC_{50}$ or $IC_{50}$ could not be obtained by fitting. | | |

**Example 8. Evaluation of anti-tumour efficacy of anti-CTLA4/TIGIT antibody in CTLA4/TIGIT double-humanised mouse model**

The calculation method used in this example is as follows:

$$\text{Tumour volume V} = 1/2 \times a \times b^2,$$

where a and b represent the length and width, respectively.

$$\text{TGI}_{TV} (\%) = [1\text{-}(Ti\text{-}T0)/(Vi\text{-}V0)] \times 100\%$$

(Ti: the mean tumour volume of the treatment group on day i of administration, T0: the mean tumour volume of the treatment group on day 0 of administration; Vi: the mean tumour volume of the vehicle control group on day i of administration, V0: the mean tumour volume of the vehicle control group on day 0 of administration) CR% (complete response rate) = number of mice with complete tumour regression (tumour volume < 50 mm$^3$ for at least two consecutive measurements)/number of mice enrolled.

Data statistics and analysis: Raw data from measurements and observations must be recorded. Analysis was performed on the basis of the raw data, and the results were expressed as means $\pm$ standard error of the mean (Mean $\pm$ SEM). Statistical analysis was also performed on the tumour volumes, with p < 0.05 considered statistically significant. Both statistical and biological significance were considered when the results were analysed.

1. Detection of efficacy in CTLA4/TIGIT double-humanised mouse MC38 colon cancer xenograft model

Experimental method: CTLA4/TIGIT double-humanised mice (Animal Centre of Biocytogen Pharmaceuticals (Beijing) Co., Ltd.) were acclimatised for 7 days. MC38 cells were subcutaneously inoculated on the right side of the mice at $5 \times 10^5$ cells/0.1 mL/mouse. When the average tumour volume reached 95 mm$^3$, 30 mice were selected and randomly divided into 5 groups (6 mice per group): G1: 1 $\times$ PBS, G2: Ipi ( 0.3 mg/kg), G3: Ipi ( 1 mg/kg), G4: As-3 ( 0.3 mg/kg), and G5: As-3 ( 1 mg/kg). The administration began on the day of grouping (Day 0). The route of administration was intraperitoneal injection. Administration was performed twice weekly for a total of 6 administrations. The body weights and tumour volumes of the mice were measured twice weekly during administration and observation. At the end of the experiment, the animals were euthanised, the tumours were excised and photographed, and the tumour growth inhibition rate (TGI$_{TV}$) was calculated.

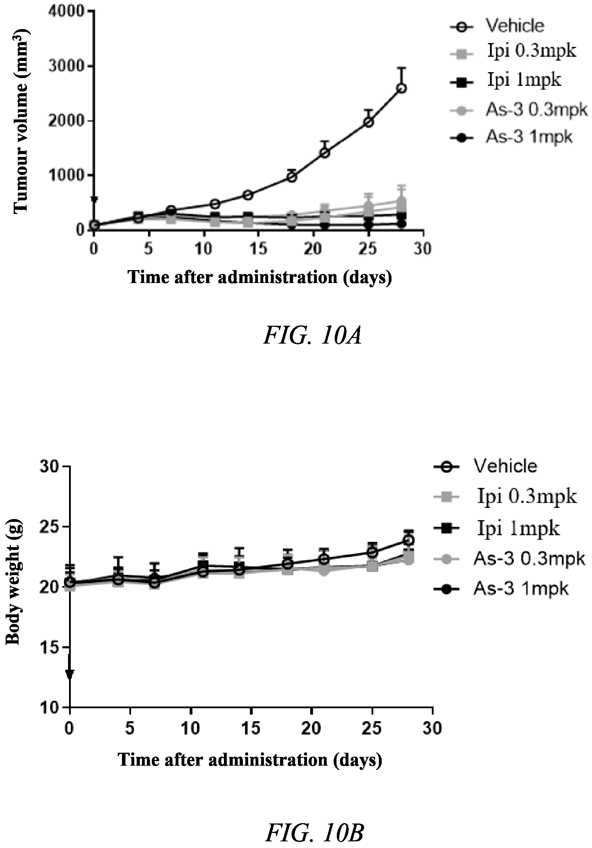

The results are shown in FIG. 10A and Table 10. The results showed that As-3 exhibited significant, dose-dependent anti-tumour activity, with the anti-tumour activity not weaker than that of Ipi and without causing significant body weight changes in the mice (FIG. 10B).

Table 10. Results of inhibitory effect of anti-CTLA4/TIGIT antibody on MC38 tumour xenografts in CTLA4-TIGIT double-humanised mice

| Group | Administration regimen | Administration dose | TGI (%) | p value |
|---|---|---|---|---|
| G1 | Blank control | - | - | - |
| G2 | Ipi | 0.3 mg/kg | 87.0 | < 0.0001 |
| G3 | Ipi | 1 mg/kg | 92.3 | < 0.0001 |
| G4 | As-3 | 0.3 mg/kg | 82.4 | 0.0003 |

(continued)

| Group | Administration regimen | Administration dose | TGI (%) | p value |
|---|---|---|---|---|
| G5 | As-3 | 1 mg/kg | 98.9 | < 0.0001 |

2. Detection of efficacy in CTLA4/TIGIT double-humanised mouse MC38 colon cancer model

Experimental method: CTLA4/TIGIT double-humanised mice (Animal Centre of Biocytogen Pharmaceuticals (Beijing) Co., Ltd.) were acclimatised for 7 days. MC38 cells were subcutaneously inoculated on the right side of B-hCTLA4/hTIGIT double-humanised mice at $5 \times 10^5$ cells/0.1 mL/mouse. When the average tumour volume reached 95 $mm^3$, the mice were randomly divided into 3 groups (3 mice per group) on the basis of tumour size: G1: $1 \times$ PBS, G2: Ipi (1 mg/kg), and G3: As-3 ( 1 mg/kg). On Day 19 and Day 21, groups G1 to G3 were administered once, for a total of 2 administrations, via intraperitoneal injection. On Day 24, anticoagulated blood (anticoagulant EDTA-K2, 150 $\mu$L/mouse) and tumours were collected from the mice in groups G1 to G3 for flow cytometry (flow cytometry panel: Live/dead, mCD45, mCD3, mCD4, mCD8, mCD25, mFoxp3). The body weights and tumour volumes of all mice in the PD group were measured twice weekly during administration and observation. At the end of the experiment, the animals were euthanised, and the tumours were excised and photographed.

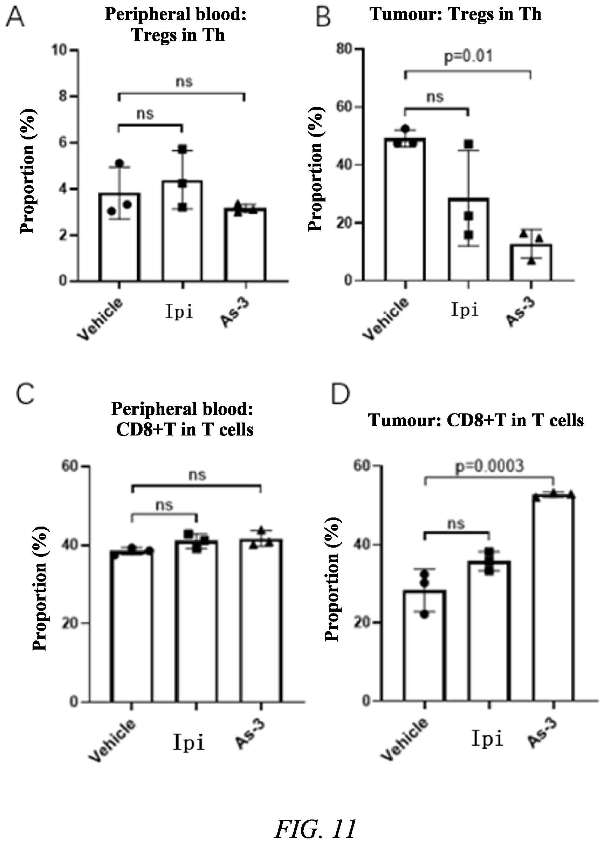

Experimental results: see FIG. 11, and Table 11. As-3 selectively depleted intratumoural Tregs, with a stronger effect than that of Ipi (FIG. 11B), while showing no significant inhibitory effect on peripheral Tregs (FIG. 11A); and As-3 induced an increase in intratumoural CD8+ T cells, with a stronger effect than that of Ipi (FIG. 11D), while causing no significant increase in peripheral CD8+ T cells (FIG. 11C).

Table 11. Results of effect of anti-CTLA4/TIGIT antibody on intratumoural and peripheral Tregs and CD8+ T cells in CTLA4-TIGIT double-humanised mice harbouring MC38 tumour xenografts

| Group | Administration regimen | Peripheral blood (% mean) | | Tumour (% mean) | |
|---|---|---|---|---|---|
| | | Tregs in Th cells | CD8+ T cells in T cells | Tregs in Th cells | CD8+ T cells in T cells |
| G1 | Blank control | 3.83% | 38.47% | 49.13% | 28.33% |
| G2 | Ipi | 4.39% | 41.03% | 28.50% | 35.80% |
| G3 | As-3 | 3.16% | 41.67% | 12.84% | 52.77% |

**Claims**

1.  A CTLA4/TIGIT binding protein, comprising:

a first binding domain that specifically binds to CTLA4, and
a second binding domain that specifically binds to TIGIT,
wherein the first binding domain that specifically binds to CTLA4 comprises a heavy chain variable region (VH1) and a light chain variable region (VL1), the VH1 comprising HCDR1, HCDR2, and HCDR3 in an amino acid sequence as set forth in SEQ ID NO: 13, and the VL1 comprising LCDR1, LCDR2, and LCDR3 in an amino acid sequence as set forth in SEQ ID NO: 14,
the second binding domain that specifically binds to TIGIT comprises a heavy chain variable region (VH2) and a light chain variable region (VL2), the VH2 comprising HCDR1, HCDR2, and HCDR3 in an amino acid sequence as set forth in SEQ ID NO: 15, and the VL2 comprising LCDR1, LCDR2, and LCDR3 in an amino acid sequence as set forth in SEQ ID NO: 16,
the CDRs are defined according to the Kabat, IMGT, Chothia, AbM, or Contact numbering system;
preferably, the VH1 comprises HCDR1, HCDR2, and HCDR3 of amino acid sequences as set forth in SEQ ID NOs: 1, 2, and 3, respectively, and the VL1 comprises LCDR1, LCDR2, and LCDR3 of amino acid sequences as set forth in SEQ ID NOs: 4, 5, and 6, respectively;
preferably, the VH2 comprises HCDR1, HCDR2, and HCDR3 of amino acid sequences as set forth in SEQ ID NOs: 7, 8, and 9, respectively, and the VL2 comprises LCDR1, LCDR2, and LCDR3 of amino acid sequences as set forth in SEQ ID NOs: 10, 11, and 12, respectively;
more preferably, the CTLA4/TIGIT binding protein is an anti-CTLA4/TIGIT antibody.

2.  The CTLA4/TIGIT binding protein according to claim 1, wherein in the first binding domain that specifically binds to CTLA4,

the VH1 comprises an amino acid sequence as set forth in SEQ ID NO: 13 or an amino acid sequence having at least 90% sequence identity thereto; and

the VL1 comprises an amino acid sequence as set forth in SEQ ID NO: 14 or an amino acid sequence having at least 90% sequence identity thereto.

3. The CTLA4/TIGIT binding protein according to claim 1 or 2, wherein in the second binding domain that specifically binds to TIGIT,

the VH2 comprises an amino acid sequence as set forth in SEQ ID NO: 15 or an amino acid sequence having at least 90% sequence identity thereto; and

the VL2 comprises an amino acid sequence as set forth in SEQ ID NO: 16 or an amino acid sequence having at least 90% sequence identity thereto.

4. The CTLA4/TIGIT binding protein according to any one of claims 1 to 3, further comprising a human immunoglobulin Fc region;

wherein preferably, the Fc region is an Fc region of IgG1, IgG2, IgG3, or IgG4;

more preferably, the Fc region is an Fc region of human IgG1, and optionally, the Fc region comprises at least one mutation of 239D, 330L, and 332E;

the mutation is defined according to the Eu numbering system.

5. The CTLA4/TIGIT binding protein according to claim 4, wherein the Fc region comprises a first subunit and a second subunit;

preferably, the first subunit of the Fc region comprises a knob mutation and the second subunit of the Fc region comprises a hole mutation.

6. The CTLA4/TIGIT binding protein according to claim 5, wherein

the Fc region comprises a first subunit comprising a mutation at position 366, and a second subunit comprising a mutation at a position selected from 366, 368, and 407, or any combination thereof; or

the Fc region comprises a first subunit comprising a mutation at position 354 or 356, and a second subunit comprising a mutation at position 349; or

the Fc region comprises a first subunit comprising a mutation at position 354 or 356, and a second subunit comprising mutations at positions 349, 366, 368, and 407;

preferably,

the Fc region comprises a first subunit comprising a 366W mutation, and a second subunit comprising a mutation selected from 366S, 368A, and 407V, or any combination thereof; or

the Fc region comprises a first subunit comprising a 354C or 356C mutation, and a second subunit comprising a 349C mutation; or

the Fc region comprises a first subunit comprising 354C/366W mutations, and a second subunit comprising 349C/366S/368A/407V mutations.

7. The CTLA4/TIGIT binding protein according to any one of claims 1 to 6, further comprising a linker, wherein preferably the linker is represented by $(G_mS_n)_h$ or $(G_mQ_n)_h$ or $(GGNGT)_h$ or $(YGNGT)_h$, or $(EPKSS)_h$, wherein m and n are each independently selected from an integer of 1-8, and h is independently selected from an integer of 1-20.

8. The CTLA4/TIGIT binding protein according to any one of claims 5 to 7, comprising a first heavy chain, a first light chain, a second heavy chain, and a second light chain; wherein

the first heavy chain, from the N-terminus to the C-terminus, sequentially comprises: [VH1]-[linker 1]-[Obscurin-O chain]-[linker 3]-[first subunit of Fc region],

the first light chain, from the N-terminus to the C-terminus, sequentially comprises: [VL1]-[linker 2]-[Titin-T chain],

the second heavy chain, from the N-terminus to the C-terminus, sequentially comprises: [VH2]-[CH1]-[second subunit of Fc region], and

the second light chain, from the N-terminus to the C-terminus, sequentially comprises: [VL2]-[CL]; wherein

- represents a peptide bond,

the amino acid sequence of the Obscurin-O chain is set forth as SEQ ID NO: 17,
the amino acid sequence of the Titin-T chain is set forth as SEQ ID NO: 18,
the linker 1, linker 2, and linker 3 may be the same or different, and may independently be present or absent; preferably, the amino acid sequences of the linker 1 and linker 2 are GGGGS, and the linker 3 is absent.

9. The CTLA4/TIGIT binding protein according to any one of claims 1 to 8, comprising the following combination of polypeptide chains:

a first heavy chain comprising an amino acid sequence as set forth in SEQ ID NO: 21 or an amino acid sequence having at least 90% sequence identity thereto,
a first light chain comprising an amino acid sequence as set forth in SEQ ID NO: 22 or an amino acid sequence having at least 90% sequence identity thereto,
a second heavy chain comprising an amino acid sequence as set forth in SEQ ID NO: 23 or an amino acid sequence having at least 90% sequence identity thereto, and
a second light chain comprising an amino acid sequence as set forth in SEQ ID NO: 24 or an amino acid sequence having at least 90% sequence identity thereto.

10. A polynucleotide encoding the CTLA4/TIGIT binding protein according to any one of claims 1 to 9.

11. A vector, comprising the polynucleotide according to claim 10.

12. A host cell, comprising the polynucleotide according to claim 10 or the vector according to claim 11.

13. A pharmaceutical composition, comprising:

the CTLA4/TIGIT binding protein according to any one of claims 1 to 9, the polynucleotide according to claim 10, or the vector according to claim 11;
wherein optionally, the pharmaceutical composition further comprises one or more pharmaceutically acceptable excipients, diluents, or adjuvants.

14. A method for preparing a CTLA4/TIGIT binding protein, comprising:

culturing the host cell according to claim 12, and expressing the CTLA4/TIGIT binding protein;
wherein optionally, the method further comprises the step of isolating and/or purifying the CTLA4/TIGIT binding protein.

15. Use of the CTLA4/TIGIT binding protein according to any one of claims 1 to 9, the polynucleotide according to claim 10, the vector according to claim 11, or the pharmaceutical composition according to claim 13 in any one of the following (1) - (3):

(1) for use in treating or alleviating cancer, or in the manufacture of a medicament for treating or alleviating cancer;
(2) for use in depleting or inhibiting Treg cells, or in the manufacture of a medicament for depleting or inhibiting Treg cells;
(3) for use in activating T cells, or in the manufacture of a medicament for activating T cells;

wherein preferably, the Treg cells are intratumoural Treg cells;
preferably, the T cells are intratumoural T cells, more preferably intratumoural CD8+ T cells;
preferably, the cancer is selected from: colourectal cancer, non-small cell lung cancer, and breast cancer.

16. A method for treating or alleviating cancer, comprising the steps of:
administering to a subject a therapeutically effective amount of the CTLA4/TIGIT binding protein according to any one of claims 1 to 9, the polynucleotide according to claim 10, the vector according to claim 11, or the pharmaceutical composition according to claim 13.

*FIG. 1A*

*FIG. 1B*

*FIG. 1C*

FIG. 1D

FIG. 2

*FIG. 3A*

*FIG. 3B*

*FIG. 3C*

*FIG. 4A*

*FIG. 4B*

*FIG. 5A*

*FIG. 5B*

*FIG. 6*

*FIG. 7A*

*FIG. 7B*

*FIG. 8*

*FIG. 9*

*FIG. 10A*

*FIG. 10B*

*FIG. 11*

*FIG. 12A*

*FIG. 12B*

## INTERNATIONAL SEARCH REPORT

| International application No. |
|---|
| **PCT/CN2024/115779** |

| A. | CLASSIFICATION OF SUBJECT MATTER |
|---|---|

C07K 16/46(2006.01)i; A61K39/395(2006.01)i; A61K39/00(2006.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC:C07K,A61K,A61P

Documentation searched other than minimum documentation to the extent that such documents are included in the fields searched

Electronic data base consulted during the international search (name of data base and, where practicable, search terms used)

CNTXT, ENTXTC, VCN, VEN, PUBMED, NCBI, EBI, ISI Web of Science, CNKI, BAIDU, BING, STN: CTLA4, TIGIT, 双特异性抗体, 抗体, CD152, VSIG9, bispecific antibody, CDR, 序列1-15, sequences 1-15

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 111212658 A (SYSTIMMUNE, INC. et al.) 29 May 2020 (2020-05-29) claims 1-41, and description, paragraphs 327-332 | 1-16 |
| Y | CN 116135884 A (BIOTHEUS INC.) 19 May 2023 (2023-05-19) claims 1-20 | 1-16 |
| Y | CN 111744013 A (JIANGSU HENGRUI MEDICINE CO., LTD. et al.) 09 October 2020 (2020-10-09) description, paragraphs 41 and 297-302 | 1-16 |
| Y | CN 111744007 A (JIANGSU HENGRUI MEDICINE CO., LTD. et al.) 09 October 2020 (2020-10-09) claim 9 | 1-16 |
| Y | CN 111727056 A (MERCK SHARP & DOHME CORP.) 29 September 2020 (2020-09-29) description, paragraphs 154-155 | 1-16 |
| Y | TW 202327649 A (JIANGSU HENGRUI MEDICINE CO., LTD. et al.) 16 July 2023 (2023-07-16) claims 1-28 | 1-16 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **02 December 2024** | **10 December 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2024/115779**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/CN2024/115779** |

**Box No. II**  **Observations where certain claims were found unsearchable (Continuation of item 2 of first sheet)**

This international search report has not been established in respect of certain claims under Article 17(2)(a) for the following reasons:

1. ☑ Claims Nos.: **15 (in part), 16**
   because they relate to subject matter not required to be searched by this Authority, namely:

   Claim 15 (in part) relates to the use of the CTLA4/TIGIT-binding protein, the polynucleotide, the vector or the pharmaceutical composition for treating or alleviating cancer, the use thereof for removing or inhibiting Treg cells, and the use thereof for activating T cells, and claim 16 relates to a method for treating or alleviating cancer, both of which fall within methods for treatment of diseases as defined in PCT Rule 39.1(iv). The present report is provided on the basis of a corresponding pharmaceutical use.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/115779**

| Patent document cited in search report | | | Publication date (day/month/year) | Patent family member(s) | | | Publication date (day/month/year) |
|---|---|---|---|---|---|---|---|
| CN | 111212658 | A | 29 May 2020 | AU | 2018358138 | A1 | 23 April 2020 |
| | | | | AU | 2018358138 | B2 | 02 June 2022 |
| | | | | AU | 2018358138 | C1 | 08 December 2022 |
| | | | | SG | 11202003237 | QA | 28 May 2020 |
| | | | | EP | 3703736 | A1 | 09 September 2020 |
| | | | | EP | 3703736 | A4 | 03 November 2021 |
| | | | | WO | 2019090002 | A1 | 09 May 2019 |
| | | | | KR | 20200091382 | A | 30 July 2020 |
| | | | | RU | 2020108444 | A | 02 December 2021 |
| | | | | RU | 2020108444 | A3 | 17 March 2022 |
| | | | | NZ | 763370 | A | 25 October 2024 |
| | | | | CA | 3069238 | A1 | 09 May 2019 |
| | | | | JP | 2021501575 | A | 21 January 2021 |
| | | | | JP | 7418326 | B2 | 19 January 2024 |
| | | | | US | 2020347137 | A1 | 05 November 2020 |
| | | | | TW | 201927819 | A | 16 July 2019 |
| | | | | IL | 271346 | A | 30 January 2020 |
| CN | 116135884 | A | 19 May 2023 | None | | | |
| CN | 111744013 | A | 09 October 2020 | None | | | |
| CN | 111744007 | A | 09 October 2020 | None | | | |
| CN | 111727056 | A | 29 September 2020 | BR | 112020015915 | A2 | 15 December 2020 |
| | | | | BR | 112020015915 | A8 | 31 January 2023 |
| | | | | WO | 2019160755 | A1 | 22 August 2019 |
| | | | | AU | 2019222517 | A1 | 13 August 2020 |
| | | | | RU | 2020129075 | A | 14 March 2022 |
| | | | | MX | 2020008446 | A | 28 September 2020 |
| | | | | AU | 2023208115 | A1 | 18 January 2024 |
| | | | | KR | 20200119845 | A | 20 October 2020 |
| | | | | JP | 2024038250 | A | 19 March 2024 |
| | | | | MA | 51844 | A | 19 May 2021 |
| | | | | EP | 3752193 | A1 | 23 December 2020 |
| | | | | EP | 3752193 | A4 | 23 February 2022 |
| | | | | JP | 2021513540 | A | 27 May 2021 |
| | | | | CA | 3090996 | A1 | 22 August 2019 |
| | | | | US | 2021047409 | A1 | 18 February 2021 |
| TW | 202327649 | A | 16 July 2023 | WO | 2023040940 | A1 | 23 March 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202311110063
- CN 109535257 A

- WO 2021139758 A1

### Non-patent literature cited in the description

- *Nat Rev Immunol.*, April 2013, vol. 13 (4), 227-42
- *Nat Immunol.*, February 2019, vol. 20 (2), 218-231
- *Oncogene*, October 2015, vol. 34 (43), 5411-7
- *J Transl Med.*, 21 November 2012, vol. 10, 236
- *Trends Immunol.*, January 2017, vol. 38 (1), 20-28
- *Biomedicines*, September 2021, vol. 9 (9), 1277
- **SCHAEFER et al.** *Proceedings of the National Academy of Sciences of the United States of America*, 2011, vol. 108 (27), 11187-11192
- **MAZOR et al.** *mAbs*, 2015, vol. 7 (2), 377-389
- **LIU et al.** *Journal of Biological Chemistry*, 2015, vol. 290 (12), 7535-7562
- **LEWIS et al.** *Nature Biotechnology*, 2014, vol. 32 (2), 191-198
- **DILLON et al.** *mAbs*, 2017, vol. 9 (2), 213-230
- Antibodies: A Laboratory Manual. Cold Spring Harbor Laboratory Press
- *J. biol. chem*, 1968, vol. 243, 3558
- GenBank, NP_005205
- GenBank, NP_776160
- **HOLLIGER** ; **HUDSON**. *Nature Biotech*, 2005, vol. 23 (9), 1126-1136
- **ADAIR** ; **LAWSON**. *Drug Design Reviews-Online*, 2005, vol. 2 (3), 209-217

- **VERMA et al.** *Journal of Immunological Methods*, 1998, vol. 216, 165-181
- **JOHNSON** ; **WU**. *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- AbMTM, A Computer Program for Modeling Variable Regions of Antibodies. **MARTIN et al.** Proc Natl Acad Sci (USA). Oxford Molecular, Ltd, 1989, vol. 86, 9268-9272
- **SAMUDRALA et al.** Ab Initio Protein Structure Prediction Using a Combined Hierarchical Approach. *PROTEINS, Structure, Function and Genetics Suppl.*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **CACECI et al.** *Byte*, 1984, vol. 9, 340-362
- **WONG** ; **LOHMAN**. *Proc. Natl. Acad. Sci. USA*, 1993, vol. 90, 5428-5432
- **SAMBROOK et al.** Molecular Cloning, A Laboratory Manual. Cold Spring Harbor Laboratory
- **AUSUBEL et al.** Current Protocols in Molecular Biology. Greene Publishing Associates